# EUROPEAN PATENT APPLICATION

(11) **EP 2 518 143 A1**
(43) Date of publication of application: **31.10.2012**
(21) Application number: 10839223.4
(22) Date of filing: 13.12.2010
(51) Int. Cl.: C12N 15/09, C12Q 1/68, G01N 21/78

(54) **PROBES FOR DETECTION OF POLYMORPHISMS OF c-kit GENE, AND USE THEREOF**

(30) Priority: 24.12.2009 JP 2009293278
(71) Applicant: Arkray, Inc., Minami-ku Kyoto-shi Kyoto 601-8045 (JP)
(72) Inventor: KOMORI Mariko, Kyoto 602-0008 (JP); IGUCHI Aki, Kyoto 602-0008 (JP)
(74) Representative: Jones, Elizabeth Louise
(86) International application number: PCT/JP2010/072374
(87) International publication number: WO 2011/077990

(57) **Abstract**

The present invention provides probes that can identify different polymorphisms in a c-kit gene easily with high reliability. Any one of oligonucleotides (P1) to (P4) is used as a probe for detecting a polymorphism in a c-kit gene.
(P1) a 4- to 50-mer oligonucleotide having a base sequence complementary to a base sequence including the 105th to 108th bases in SEQ ID NO: 1 and having the base complementary to the 105th base in its 3' end region
(P2) a 7- to 50-mer oligonucleotide having a base sequence including the 102nd to 108th bases in SEQ ID NO: 1 and having the 102nd base in its 5' end region
(P3) an 8- to 50-mer oligonucleotide having a base sequence complementary to a base sequence including the 127th to 134th bases in SEQ ID NO: 1 and having the base complementary to the 134th base in its 5' end region
(P4) a 5- to 50-mer oligonucleotide having a base sequence including the 123rd to 127th bases in SEQ ID NO: 1 and having the 123rd base in its 5' end region

## Description

### Technical Field

The present invention relates to a probe for detecting a polymorphism in a c-kit gene and to the use thereof.

### Background Art

The c-kit receptor is a receptor tyrosine kinase present on a cell surface, and it binds to a stem cell factor. The c-kit gene coding for the c-kit receptor is located on human chromosome 4. Known mutations of the c-kit gene are, for example: mutation from adenine (a) to thymine (t) at the 108th base (w) in the partial sequence of the c-kit gene shown in SEQ ID NO: 1; and mutation from thymine (t) to guanine (g) or adenine (a) at the 127th base (d) in the same (Non-Patent Document 1 etc.). In the c-kit receptor, by the mutation at the 108th base, the aspartic acid (D) as the 816th amino acid is mutated to valine (V), and by the mutation at the 127th base, asparagine (N) as the 822nd amino acid is mutated to lysine (K). It has been reported that these mutations are associated with cancers such as acute myelogenous leukemia and gastrointestinal stromal tumor (GIST), as well as poor prognosis of these cancers, for example (Non-Patent Document 1 etc.). Therefore, detecting the presence or absence of these mutations, i.e., detecting polymorphisms in the c-kit gene is very important in the diagnosis of the above-described diseases, the selection of more effective treatment methods for the diseases, etc., for example.

On the other hand, as a method for detecting a polymorphism in a gene, various methods have been reported. Examples thereof include a PCR (Polymerase Chain Reaction)-RFLP (Restriction Fragment Length Polymorphism) method.

The PCR-RFLP method is carried out by amplifying a detection target region in a target DNA in a sample by PCR, treating the obtained amplification product with a restriction enzyme, and typing the change in restriction fragment length caused by a polymorphism according to Southern hybridization. When a target mutation is present in the gene, the recognition site of the restriction enzyme disappears. Thus, it is possible to detect the presence or absence of the mutation based on the presence or absence of cleavage, i.e., the change in restriction fragment length.

However, in the PCR-RFLP method, for example, after the PCR, it is necessary to conduct a cumbersome procedure of treating the obtained amplification product with a restriction enzyme and conducting an analysis. Furthermore, in order to treat the obtained amplification product with a restriction enzyme, the amplification product has to be temporarily taken out from a reactor. Thus, there is a risk that the amplification product obtained in a first reaction may be dispersed and mixed with a second reaction that is different from the first reaction. Such problems make the automation of polymorphism detection difficult.

In light of these problems, Tm (Melting Temperature) analysis is attracting attention as a method for detecting a polymorphism in recent years. Tm analysis also is referred to as a melting curve analysis. In Tm analysis, first, using a probe complementary to a region including a detection target polymorphism, a hybrid (double-stranded nucleic acid) of a nucleic acid to be examined (hereinafter simply referred to as a "test nucleic acid") with the probe is formed. Then, the thus-obtained hybrid is heat-treated, and dissociation (melting) of the hybrid into single-stranded nucleic acids accompanying the temperature rise is detected by measuring a signal such as absorbance. By determining the Tm value based on the result of the detection, the polymorphism is determined. The Tm value becomes higher as the complementarity between the single-stranded nucleic acids of the hybrid becomes higher, and becomes lower as the complementarity between the same becomes lower. Thus, in the case where the polymorphism in a detection target site is X or Y, the Tm value of a hybrid of a nucleic acid containing the target polymorphism (e.g., Y) and a probe that is 100% complementary thereto is determined beforehand (this Tm value represents the evaluation standard value). Subsequently, the Tm value of a hybrid of the test nucleic acid and the probe is measured (this Tm value represents the measured value). Then, when this measured value is the same as the evaluation standard value, it can be determined that the test nucleic acid shows a perfect match with the probe, i.e., the detection target site in the test nucleic acid is the target polymorphism (Y). On the other hand, when the measured value is lower than the evaluation standard value, it can be determined that the test nucleic acid shows a mismatch with the probe, i.e., the detection target site in the test nucleic acid is the other polymorphism (X). According to such a method, a polymorphism can be detected merely by heat-treating a PCR reaction solution containing the probe and then measuring the signal, for example. Thus, it is possible to automate a detection device.

However, in detection methods utilizing such Tm analysis, it is necessary to determine the difference in a single base from the Tm value, for example. Furthermore, when the gene has a plurality of polymorphisms, a significant amount of labour is required to analyze even a single sample. Thus, there is a problem in that analyzing a large number of samples is not practical. Therefore, there is a demand for a detection method that can detect the presence or absence of mutation accurately, especially even in the case where a wild-type polymorphism and a mutant polymorphism are present together, for example.

### Citation List

### Non-Patent Document(s)

[Non-Patent Document 1] J Clin Oncol., August 20, 2006, Vol. 24, No. 24, pp. 3904-3911

### Brief Summary of the Invention

### Problem to be Solved by the Invention

For the reasons stated above, detecting a polymorphism in a c-kit gene is very important in the diagnosis of the above-described diseases and the selection of the treatment methods for the diseases, for example. With the foregoing in mind, it is an object of the present invention to provide a probe that can identify a polymorphism in a c-kit gene easily with high reliability, and use of the probe.

### Means for Solving Problem

In order to achieve the above object, the present invention provides a probe for detecting a polymorphism in a c-kit gene, including: any one of the following oligonucleotides (P1) to (P4) and (P1') to (P4'):
(P1) a 4- to 50-mer oligonucleotide having a base sequence complementary to a base sequence including the 105th to 108th bases in SEQ ID NO: 1 and having the base complementary to the 105th base in its 3' end region;
(P1') an oligonucleotide having a base sequence complementary to oligonucleotide (P1);
(P2) a 7- to 50-mer oligonucleotide having a base sequence including the 102nd to 108th bases in SEQ ID NO: 1 and having the 102nd base in its 5' end region;
(P2') an oligonucleotide having a base sequence complementary to oligonucleotide (P2);
(P3) an 8- to 50-mer oligonucleotide having a base sequence complementary to a base sequence including the 127th to 134th bases in SEQ ID NO: 1 and having the base complementary to the 134th base in its 5' end region;
(P3') an oligonucleotide having a base sequence complementary to oligonucleotide (P3);
(P4) a 5- to 50-mer oligonucleotide having a base sequence including the 123rd to 127th bases in SEQ ID NO: 1 and having the 123rd base in its 5' end region; and
(P4') an oligonucleotide having a base sequence complementary to oligonucleotide (P4).

The present invention also provides a method for detecting a polymorphism in a c-kit gene, including the step of: detecting a polymorphism in a c-kit gene using the probe according to the present invention.

The present invention also provides a reagent for detecting a polymorphism in a c-kit gene, containing the probe according to the present invention.

### Effects of the Invention

According to the probe of the present invention, a polymorphism in a c-kit gene can be identified easily with high reliability by Tm analysis, for example. Specifically, for example, even in the case where a c-kit gene having a wild-type target polymorphism and a c-kit gene having a mutant target polymorphism are present together in a sample, the kind of polymorphism or the presence or absence of mutation can be detected easily with high reliability by Tm analysis using the probe of the present invention. Therefore, the present invention is particularly useful when applied to a sample containing both a wild-type c-kit gene and a mutant c-kit gene, for example. Thus, according to the present invention, a polymorphism in a c-kit gene can be identified easily with high reliability, so that, for example, the detection result can be reflected in the diagnosis of the above-described diseases, selection of the treatment methods for the diseases, and the like. Hence, it can be said that the present invention is very useful in the medical field and the like.

### Brief Description of Drawings

[FIG. 1] FIG. 1 shows graphs each showing the result of Tm analysis with respect to a reaction solution containing wild-type and mutant plasmids in Example 1 of the present invention.
[FIG. 2] FIG. 2 shows graphs each showing the result of Tm analysis with respect to a reaction solution containing wild-type and mutant plasmids in Example 2 of the present invention.
[FIG. 3] FIG. 3 shows graphs each showing the result of Tm analysis with respect to a reaction solution containing wild-type and mutant plasmids in Example 3 of the present invention.

### Mode for Carrying out the Invention

In the present invention, detection target polymorphisms in a c-kit gene are, for example, polymorphisms at the 108th base (w) and the 127th base (d) in the partial sequence of the c-kit gene shown in SEQ ID NO: 1. The 108th base (w) is adenine (a) in the wild-type c-kit gene. In this case, the 816th amino acid in the c-kit receptor is an aspartic acid (D) (D816). The 108th base (w) is thymine (t) in a mutant c-kit gene. In this case, the 816th amino acid in the c-kit receptor is valine (V) (D816V). The 127th base (d) is thymine (t) in the wild-type c-kit gene. In this case, the 822nd amino acid in the c-kit receptor is asparagine (N) (N822). The 127th base (d) is guanine (g) or adenine (a) in a mutant c-kit gene. In these cases, the 822nd amino acid in the c-kit receptor is lysine (K) (N822K). When at least one of the two polymorphisms is a mutant in a c-kit gene, it can be determined that the subject has a possibility of a cancer such as acute myelogenous leukemia or gastrointestinal stromal tumor (GIST) or a possibility of poor prognosis of the above-described disease, for example.

Hereinafter, in the c-kit gene, the polymorphisms in the codon corresponding to the 816th amino acid are referred to as "816 polymorphisms". Specifically, the polymorphism that results in D816 also is referred to as a "D816 wild-type or gac wild-type", and the polymorphism that results in D816V also is referred to as a "D816V mutant or gtc mutant". Hereinafter, in the c-kit gene, the polymorphisms in the codon corresponding to the 822nd amino acid are referred to as "822 polymorphisms". Specifically, the polymorphism that results in N822 also is referred to as an "N822 wild-type or aat wild-type", and the polymorphism that results in N822K also is referred to as an "N822K mutant, aag mutant, or aaa mutant".

The base sequence of the c-kit gene is registered under GenBank Accession No. NG_007456 as a sequence from the 4935th to 87721st bases, for example. The base sequence of SEQ ID NO: 1 is a partial sequence of the c-kit gene in the base sequence of Accession No. NG_007456, and it corresponds to a region from the 80054th to 80293rd bases in the base sequence of Accession No. NG_007456.

In the present invention, hereinafter, a c-kit gene of D816 wild-type also is referred to as the "D816 wild-type gene", and a c-kit gene of D816V mutant also is referred to as the "D816V mutant gene". Hereinafter, a c-kit gene of N822 wild-type also is referred to as the "N822 wild-type gene", and a c-kit gene of N822K mutant also is referred to as the "N822K mutant gene".

In the present invention, the sites at which the above-described polymorphisms occur, i.e., the 108th and 127th bases in the base sequence of SEQ ID NO: 1 (sense strand) or bases to be paired with the 108th and 127th bases in the sense strand of a sequence complementary thereto (antisense strand) are referred to as "detection target sites". In the base sequence of SEQ ID NO: 1 (sense strand) or in the sequence complementary thereto (antisense strand), a region including any of the detection target sites and to which the probe can hybridize is referred to as a "hybridization region or detection target sequence". The detection target sequence in which the detection target site is wild-type also is referred to as a "wild-type detection target sequence". The detection target sequence in which the detection target site is mutant also is referred to as a "mutant detection target sequence". Among the above-described detection target sequences, the one showing a perfect match with the polymorphism detection probe is referred to as a "perfect match sequence", and the one showing a mismatch with the polymorphism detection probe is referred to as a "mismatch sequence". In the present invention, the term "perfect match" means that the base at the detection target site is complementary to the base to be paired therewith in the polymorphism detection probe, and preferably means that the detection target sequence is perfectly complementary with the polymorphism detection probe. In the present invention, the term "mismatch" means that the base at the detection target site is not complementary to the base to be paired therewith in the polymorphism detection probe, and preferably means that the detection target sequence is perfectly complementary to the polymorphism detection probe except for the detection target site.

In the present invention, a probe for detecting the 816 polymorphism (a/t) also is referred to as an "816 probe", and a probe for detecting the 822 polymorphism (t/g, t/a) also is referred to as an "822 probe". A probe that shows a perfect match with the wild-type detection target site in the detection target sequence also is referred to as a "wild-type probe", and a probe that shows a perfect match with the mutant detection target site in the detection target sequence also is referred to as a "mutant probe".

In the present invention, in the case where a c-kit gene is amplified and further, the probe of the present invention is caused to hybridize with the obtained amplification product, a region to be amplified in the c-kit gene hereinafter is referred to as an "amplification target region". The amplification target region may be, for example, a region in the sense strand of the c-kit gene, a region corresponding thereto in the antisense strand, or both of them. In the present invention, the terms "sense strand" and "antisense strand" encompass, for example, amplification products of the sense strand and amplification products of the antisense strand, respectively.

In the present invention, the ends of a base sequence means the endmost bases on the 5' side and the 3' side in the base sequence. Furthermore, a 5' end region is a region including several bases from the 5' end in a base sequence, and a 3' end region is a region including several bases from the 3' end in a base sequence. The several bases mean, for example, 1 to 10, 1 to 4, 1 to 3, or 1 to 2 bases counted from the end. In the present invention, the Zth base (Z is a positive integer) from an end of a base sequence is a numerical order counted with the base at the end as the first base. For example, the first base from the end means the base at the end, and the second base from the end means a base next to the base at the end.

### <Polymorphism Detection Probe>

The polymorphism detection probe of the present invention is, as described above, a probe for detecting a polymorphism in a c-kit gene, including: any one of the following oligonucleotides (P1) to (P4) and (P1') to (P4'):
(P1) a 4- to 50-mer oligonucleotide having a base sequence complementary to a base sequence including the 105th to 108th bases in SEQ ID NO: 1 and having the base complementary to the 105th base in its 3' end region;
(P1') an oligonucleotide having a base sequence complementary to oligonucleotide (P1);
(P2) a 7- to 50-mer oligonucleotide having a base sequence including the 102nd to 108th bases in SEQ ID NO: 1 and having the 102nd base in its 5' end region;
(P2') an oligonucleotide having a base sequence complementary to oligonucleotide (P2);
(P3) an 8- to 50-mer oligonucleotide having a base sequence complementary to a base sequence including the 127th to 134th bases in SEQ ID NO: 1 and having the base complementary to the 134th base in its 5' end region;
(P3') an oligonucleotide having a base sequence complementary to oligonucleotide (P3);
(P4) a 5- to 50-mer oligonucleotide having a base sequence including the 123rd to 127th bases in SEQ ID NO: 1 and having the 123rd base in its 5' end region; and
(P4') an oligonucleotide having a base sequence complementary to oligonucleotide (P4).

The base lengths of the oligonucleotides (P1) and (P1') are 4- to 50-mer, preferably 10- to 50-mer, more preferably 13- to 30-mer, and still more preferably 15- to 20 -mer. The base lengths of the oligonucleotides (P2) and (P2') are 7- to 50-mer, preferably 10- to 50-mer, more preferably 13- to 30-mer, and still more preferably 15- to 20-mer. The base lengths of the oligonucleotides (P3) and (P3') are 8- to 50-mer, preferably 10- to 50-mer, more preferably 13- to 30-mer, and still more preferably 15- to 20 -mer. The base lengths of the oligonucleotides (P4) and (P4') are 5- to 50-mer, preferably 10- to 50-mer, more preferably 13- to 30-mer, and still more preferably 15- to 20-mer.

A probe including any one of the oligonucleotides (P1), (P1'), (P2), and (P2') is a probe for detecting the 816 polymorphisms (a/t), i.e., the 816 probe. In the base sequence of SEQ ID NO: 1, the 108th base (w) is adenine (a) or thymine (t), and the 127th base (d) is thymine (t), guanine (g), or adenine (a) (hereinafter the same).

The oligonucleotide (P1) is complementary to the sense strand of the c-kit gene, for example, and the polymorphism can be checked through the hybridization thereof with the sense strand.

In the oligonucleotide (P1), a complementary base to be paired with the 108th base (w = a, t) in SEQ ID NO: 1 is represented by "w", which is thymine (t) or adenine (a). The oligonucleotide in which w is thymine (t) shows a perfect match with a detection target sequence including the D816 wild-type detection target site. Thus, the oligonucleotide can be referred to as a D816 wild-type probe. The oligonucleotide in which w is adenine (a) shows a perfect match with a detection target sequence including the D816V mutant detection target site. Thus, the oligonucleotide can be referred to as a D816V mutant probe. The 816 polymorphisms in the c-kit gene can be detected based on whether or not these oligonucleotides show a perfect match with the detection target sequences in the c-kit gene.

The oligonucleotide (P1) has the base complementary to the 105th base in its 3' end region. Preferably, the base is located at a position of the 1st to 4th bases from its 3' end, more preferably the 1st to 3rd bases from its 3' end, and particularly preferably the 1st base (3' end) or the 2nd base from its 3' end.

Examples of the oligonucleotide (P1) include an oligonucleotide of SEQ ID NO: 2, in which w is thymine or adenine. A specific example of this oligonucleotide is an oligonucleotide of SEQ ID NO: 6, for example. A probe including the oligonucleotide of SEQ ID NO: 6 can be used as a D816V mutant probe, for example.
5'-aatcattcttgatgwctc-3' (SEQ ID NO: 2)
5'-aatcattcttgatgactc-3' (SEQ ID NO: 6)

As described above, the oligonucleotide (P1') is complementary to the oligonucleotide (P1). The oligonucleotide (P1') also can be defined as follows, for example.
(P1') a 4- to 50-mer oligonucleotide having a base sequence including the 105th to 108th bases in SEQ ID NO: 1 and having the 105th base in its 5' end region

The oligonucleotide (P1') is homologous to the sense strand of the c-kit gene, and the polymorphism can be checked through the hybridization thereof with the antisense strand.

The oligonucleotide (P2) is homologous to the sense strand of the c-kit gene, for example, and the polymorphism can be checked through the hybridization thereof with the antisense strand.

In the oligonucleotide (P2), the 108th base (w) in SEQ ID NO: 1 is, as described above, adenine (a) or thymine (t). The oligonucleotide in which w is adenine (a) shows a perfect match with a detection target sequence including the D816 wild-type detection target site. Thus, the oligonucleotide can be referred to as a D816 wild-type probe. The oligonucleotide in which w is thymine (t) shows a perfect match with a detection target sequence including the D816V mutant detection target site. Thus, the oligonucleotide can be referred to as a D816V mutant probe. The 816 polymorphisms in the c-kit gene can be detected based on whether or not these oligonucleotides show a perfect match with the detection target sequences of the c-kit gene.

The oligonucleotide (P2) has the 102nd base in its 5' end region. Preferably, the base is located at a position of the 1st to 4th bases from its 5' end, more preferably the 1st to 3rd bases from its 5' end, and particularly preferably the 1st base (5' end) or the 2nd base from its 5' end.

Examples of the oligonucleotide (P2) include the oligonucleotide of SEQ ID NO: 3, in which w is thymine or adenine. A specific example of this oligonucleotide is the oligonucleotide of SEQ ID NO: 7, for example. A probe including the oligonucleotide of SEQ ID NO: 7 can be used as a D816V mutant probe, for example.
5'-ccagagwcatcaagaatg-3' (SEQ ID NO: 3)
5'-ccagagtcatcaagaatg-3' (SEQ ID NO: 7)

As described above, the oligonucleotide (P2') is complementary to the oligonucleotide (P2). The oligonucleotide (P2') also can be defined as follows, for example.
(P2') a 7- to 50-mer oligonucleotide having a base sequence complementary to a base sequence including the 102nd to 108th bases in SEQ ID NO: 1 and having the base complementary to the 102nd base in its 3' end region

The oligonucleotide (P2') is complementary to the sense strand of the c-kit gene, and the polymorphism can be checked through the hybridization thereof with the sense strand.

A probe including any one of the oligonucleotides (P3), (P3'), (P4), and (P4') is a probe for detecting the 822 polymorphisms (t/g, t/a), i.e., the 822 probe. In the base sequence of SEQ ID NO: 1, the 127th base (d) is thymine (t), guanine (g), or adenine (a) (hereinafter the same).

The oligonucleotide (P3) is complementary to the sense strand of the c-kit gene, for example, and the polymorphism can be checked through the hybridization thereof with the sense strand.

In the oligonucleotide (P3), a complementary base to be paired with the 127th base (d = t, g, a) in SEQ ID NO: 1 is represented by "h", which is adenine (a), cytosine (c), or thymine (t). The oligonucleotide in which h is adenine (a) shows a perfect match with a detection target sequence including the N822 wild-type detection target site. Thus, the oligonucleotide can be referred to as an N822 wild-type probe. The oligonucleotide in which h is cytosine (c) shows a perfect match with a detection target sequence including the N822K mutant detection target site. Thus, the oligonucleotide can be referred to as an N822K mutant probe. The oligonucleotide in which h is thymine (t) shows a perfect match with a detection target sequence including the N822K mutant detection target site. Thus, the oligonucleotide can be referred to as an N822K mutant probe. The 822 polymorphisms of the c-kit gene can be detected based on whether or not these oligonucleotides show a perfect match with the detection target sequences of the c-kit gene.

The oligonucleotide (P3) has the base complementary to the 134th base in its 5' end region. Preferably, the base is located at a position of the 1st to 4th bases from its 5' end, more preferably the 1st to 3rd bases from its 5' end, and particularly preferably the 1st (5' end) or the 2nd base from its 5' end.

Examples of the oligonucleotide (P3) include the oligonucleotide shown in SEQ ID NO: 4, in which h is adenine (a), cytosine (c), or thymine (t). A specific example of this oligonucleotide is the oligonucleotide of SEQ ID NO: 8, for example. A probe including the oligonucleotide of SEQ ID NO: 8 can be used as an N822K mutant probe, for example.
5'-ccacatahttagaatcattctt-3' (SEQ ID NO: 4)
5'-ccacatacttagaatcattctt-3' (SEQ ID NO: 8)

As described above, the oligonucleotide (P3') is complementary to the oligonucleotide (P3). The oligonucleotide (P3') also can be defined as follows, for example.
(P3') an 8- to 50-mer oligonucleotide having a base sequence including the 127th to 134th bases in SEQ ID NO: 1 and having the 134th base in its 3' end region

The oligonucleotide (P3') is homologous to the sense strand of the c-kit gene, and the polymorphism can be checked through the hybridization thereof with the antisense strand.

The oligonucleotide (P4) is homologous to the sense strand of the c-kit gene, for example, and the polymorphism can be checked through the hybridization thereof with the antisense strand.

In the oligonucleotide (P4), the 127th base (d) in SEQ ID NO: 1 is, as described above, thymine (t), guanine (g), or adenine (a). The oligonucleotide in which d is thymine (t) shows a perfect match with a detection target sequence including the N822 wild-type detection target site. Thus, the oligonucleotide can be referred to as an N822 wild-type probe. The oligonucleotide in which d is guanine (g) shows a perfect match with a detection target sequence including the N822K mutant detection target site. Thus, the oligonucleotide can be referred to as an N822K mutant probe. The oligonucleotide in which d is adenine (a) shows a perfect match with a detection target sequence including the N822K mutant detection target site. Thus, the oligonucleotide can be referred to as an N822K mutant probe. The 822 polymorphisms of the c-kit gene can be detected based on whether or not these oligonucleotides show a perfect match with the detection target sequences of the c-kit gene.

The oligonucleotide (P4) has the 123rd base in its 5' end region. Preferably, the base is located at a position of the 1st to 4th bases from its 5' end, more preferably the 1st to 3rd bases from its 5' end, and particularly preferably the 1st (5' end) or the 2nd base from its 5' end.

Examples of the oligonucleotide (P4) include the oligonucleotide shown in SEQ ID NO: 5, in which d is thymine (t), guanine (g), or adenine (a). A specific example of this oligonucleotide is the oligonucleotide shown in SEQ ID NO: 9, for example. A probe including the oligonucleotide of SEQ ID NO: 9 can be used as an N822K mutant probe, for example.
5'-ctaadtatgtggttaaaggaaa-3' (SEQ ID NO: 5)
5'-ctaagtatgtggttaaaggaaa-3' (SEQ ID NO: 9)

As described above, the oligonucleotide (P4') is complementary to the oligonucleotide (P4). The oligonucleotide (P4') also can be defined as follows, for example.
(P4') a 5- to 50-mer oligonucleotide having a base sequence complementary to a base sequence including the 123rd to 127th bases in SEQ ID NO: 1 and having the base complementary to the 123rd base in its 3' end region

The oligonucleotide (P4') is complementary to the sense strand of the c-kit gene, and the polymorphism can be checked through the hybridization thereof with the sense strand.

The probe of the present invention may be a probe including any one of the above-described oligonucleotides or a probe consisting of any one of the above-described oligonucleotides, for example.

The polymorphism detection probe of the present invention may be configured as follows. For example, the oligonucleotide (P1) or (P3) may be such that it has a base sequence derived from the above base sequence by deletion, substitution, or addition of one or more bases at any site other than the base to be paired with the detection target site and the base complementary to the 105th or 134th base and can hybridize to the detection target sequence. Also, for example, the oligonucleotide (P1') or (P3') may be such that it has a base sequence derived from the above base sequence by deletion, substitution, or addition of one or more bases at any site other than the base to be paired with the detection target site and the base complementary to the 105th or 134th base and can hybridize to the detection target sequence. Also, for example, the oligonucleotide (P2) or (P4) may be such that it has a base sequence derived from the above base sequence by deletion, substitution, or addition of one or more bases at any site other than the base to be paired with the detection target site and the base complementary to the 102nd or 123rd base and can hybridize to the detection target sequence. Also, for example, the oligonucleotide (P2') or (P4') may be such that it has a base sequence derived from the above base sequence by deletion, substitution, or addition of one or more bases at any site other than the base to be paired with the detection target site and the base complementary to the 102nd or 123rd base and can hybridize to the detection target sequence.

Any one kind of the probe may be used, or two or more kinds of them may be used in combination, for example. When two or more kinds are used in combination, it is preferable to use a wild-type probe and a mutant probe in combination, for example. With this configuration, it becomes possible not only to detect whether the polymorphism is wild-type or mutant but also to identify the mutant.

The probe of the present invention preferably is a labeled probe having a labeling substance. For example, it is preferable that the oligonucleotide is labeled (modified) with the labeling substance. In the oligonucleotide, a site to be labeled with the labeling substance is not particularly limited, and preferably it is the 5' end region or the 3' end region, more preferably the 5' end or the 3' end, for example. As will be described below, in the oligonucleotide, a base to be labeled with the labeling substance preferably is cytosine (c) or guanine (g), for example. The base may be labeled directly with the labeling substance, or alternatively, it may be labeled indirectly with the labeling substance, for example. In the latter case, for example, by labeling any site in a nucleotide residue containing the base, the base can be labeled indirectly with the labeling substance. In the oligonucleotide (P1), it is preferable that cytosine (c) at the 3' end is labeled with the labeling substance, for example. In each of the oligonucleotides (P2) to (P4), it is preferable that cytosine (c) at the 5' end is labeled with the labeling substance, for example. In the oligonucleotide (P1'), it is preferable that guanine (g) at the 5' end is labeled with the labeling substance, for example. In each of the oligonucleotides (P2') to (P4'), it is preferable that guanine (g) at the 3' end is labeled with the labeling substance, for example.

It is preferable that each of the oligonucleotides (P1), (P2'), (P3'), and (P4') has the labeling substance in its 3' end region. Specifically, it is preferable that the labeling substance is located at a position of the 1st to 4th bases from its 3' end, more preferably the 1st to 3rd bases from its 3' end, and particularly preferably the 2nd base from its 3' end or the base at its 3' end, for example. In the oligonucleotide (P1), it is preferable that the base complementary to any of the 102nd to 108th bases, more preferably the base complementary to the 105th base has the labeling substance, for example. In the oligonucleotide (P2'), it is preferable that the base complementary to any of the 99th to 105th bases, more preferably the base complementary to the 102nd base has the labeling substance, for example. In the oligonucleotide (P3'), it is preferable that any of the 131st to 137th bases, more preferably the 134th base has the labeling substance, for example. In the oligonucleotide (P4'), it is preferable that the base complementary to any of the 120th to 126th bases, more preferably the base complementary to the 123rd base has the labeling substance, for example.

It is preferable that each of the oligonucleotides (P1'), (P2), (P3), and (P4) has the labeling substance in its 5' end region. Specifically, it is preferable that the labeling substance is located at a position of the 1st to 4th bases from its 5' end, more preferably the 1st to 3rd bases from its 5' end, and particularly preferably the 2nd base from its 5' end or the base at its 5' end, for example. In the oligonucleotide (P1'), it is preferable that any of the 102nd to 108th bases, more preferably the 105th base has the labeling substance, for example. In the oligonucleotide (P2), it is preferable that any of the 99th to 105th bases, more preferably the 102nd base has the labeling substance, for example. In the oligonucleotide (P3), it is preferable that the base complementary to any of the 131st to 137th bases, more preferably the base complementary to the 134th base has the labeling substance, for example. In the oligonucleotide (P4), it is preferable that any of the 120th to 126th bases, more preferably the 123rd base has the labeling substance, for example.

The labeling substance is not particularly limited, and preferably is the one that gives off a signal depending on whether the labeled probe is present independently or it forms a hybrid, for example. The kind of signal is not particularly limited, and examples of the signal include fluorescence, colouring, and colour development. When the signal is fluorescence, examples of a signal value include fluorescence intensity. When the signal is colouring or colour development, examples of a signal value include reflectance, absorbance, and transmittance. The signal may be given off from the labeling substance directly or indirectly, for example.

The labeling substance is not particularly limited, and examples thereof include fluorescent substances such as a fluorophore. Examples of the fluorescent substance include fluorescein, phosphor, rhodamine, and polymethine dye derivatives. Examples of commercially available fluorescent substances include Pacific Blue® (Molecular Probes), BODIPY FL® (Molecular Probes), FluorePrime™ (Amersham Pharmacia), Fluoredite™ (Millipore Corporation), FAM® (ABI), Cy3™ and Cy5™ (Amersham Pharmacia), and TAMRA® (Molecular Probes). The detection conditions for the fluorescent substance are not particularly limited, and can be determined as appropriate depending on the kind of fluorescent substance to be used, for example. For example, Pacific Blue can be detected at a detection wavelength from 450 to 480 nm; TAMRA can be detected at a detection wavelength from 585 to 700 nm; and BODIPY FL can be detected at a detection wavelength from 515 to 555 nm. When such a probe is used, for example, by detecting fluorescence as the signal and measuring fluorescence intensity as the signal value, hybridization and dissociation can be checked easily based on the change in fluorescence intensity.

Preferably, the labeled probe is a labeled probe that shows a signal independently and shows no signal when it forms a hybrid, or a labeled probe that shows no signal independently and shows a signal when it forms a hybrid, for example. When the labeling substance is a fluorescent substance, the labeled probe preferably is a probe that is labeled with the fluorescent substance, shows fluorescence independently, and shows reduced (e.g., quenched) fluorescence when it forms a hybrid, for example. Such a phenomenon generally is called fluorescence quenching. Probes utilizing this phenomenon generally are called fluorescence quenching probes. Among these fluorescence quenching probes, a preferred probe is one in which the 3' end or the 5' end of the oligonucleotide is labeled with the fluorescent substance, and the base at the end to be labeled preferably is cytosine (c) or guanine (g). In the case where the base at the end is cytosine (c), the base sequence of the fluorescence quenching probe preferably is designed so that, for example, when the fluorescence quenching probe forms a hybrid with a test nucleic acid, the base to be paired with the labeled cytosine (c) at the end or a base away therefrom by one to three bases in the test nucleic acid is guanine (g). A base away from the base to be paired with cytosine (c) by one base is a base located next to the base to be paired with cytosine (c). Such a probe generally is called a guanine quenching probe, and is known as a so-called QProbe®. When such a guanine quenching probe hybridizes to the test nucleic acid, there occurs a phenomenon that, for example, as the fluorescent substance-labeled cytosine (c) at the end approaches guanine (g) in the test nucleic acid, fluorescence of the fluorescent substance becomes weak (the fluorescence intensity is reduced). By using the probe such as described above, hybridization and dissociation can be checked easily based on the change in fluorescence intensity. Similarly, in the case where the above-described base at the end is guanine (g), the base sequence of the fluorescence quenching probe preferably is designed so that, for example, when the fluorescence quenching probe forms a hybrid with a test nucleic acid, the base to be paired with the labeled guanine (g) at the end or a base away therefrom by one to three bases in the test nucleic acid is cytosine (c).

In the polymorphism detection probe of the present invention, for example, a phosphate group may be added to the 3' end. As will be described below, a test nucleic acid can be prepared by a nucleic acid amplification method such as PCR, for example. At this time, it is possible to add the polymorphism detection probe of the present invention to a reaction system of the nucleic acid amplification reaction. In such a case, if the 3' end of the probe has a phosphate group added thereto, it is possible to sufficiently prevent the polymorphism detection probe itself from being elongated by the nucleic acid amplification reaction. A similar effect is obtained also by adding a labeling substance such as described above to the 3' end.

In the detection of a polymorphism using the polymorphism detection probe of the present invention, the detection method is by no means limited as long as it is a method utilizing the hybridization of the probe with the detection target sequence. As the detection method for a polymorphism, the polymorphism detection method according to the present invention will be described below.

### <Polymorphism Detection Method>

The polymorphism detection method according to the present invention is, as described above, a method for detecting a polymorphism in a c-kit gene, including the step of: detecting a polymorphism in a c-kit gene using the probe for detecting a polymorphism in a c-kit gene according to the present invention.

The polymorphism detection method of the present invention preferably includes the steps of:
(A) while changing the temperature of a reaction system containing the probe of the present invention and a test nucleic acid including the polymorphism to be detected, measuring a signal value indicating the melting state of a hybrid of the test nucleic acid and the probe; and
(B) detecting the polymorphism in the test nucleic acid based on the change in signal value accompanying the temperature change.

The polymorphism detection method of the present invention is characterized in that it uses the probe of the present invention, and other configurations, conditions, and the like are not limited to those described below. As described above, the probe of the present invention preferably is a labeled probe. In the present invention, the reaction system is a reaction solution, for example.

In the present invention, any one kind of polymorphism detection probe of the present invention may be used, or two or more kinds may be used in combination, for example. The kind of probe to be used can be determined as appropriate depending on, for example, the detection target polymorphism.

In the case where the polymorphism detection probe is used, it is only necessary that at least one kind of polymorphism detection probe is the probe of the present invention. The kind of probe can be determined as appropriate depending on the detection target polymorphism in the c-kit gene, for example. In the present invention, for example, only the 816 polymorphism or only the 822 polymorphism may be detected. Also, it is possible to detect both the polymorphisms in a single reaction system. Moreover, it is also possible to detect at least one of the 816 polymorphism and 822 polymorphism and any other polymorphism in a single reaction system.

When only the 816 polymorphism is to be detected, it is preferable to use the 816 probe among the polymorphism detection probes of the present invention. The 816 probe may be either the D816 wild-type probe or the D816V mutant probe, or both of them may be used in combination, for example. When only the 822 polymorphism is to be detected, it is preferable to use the 822 probe among the polymorphism detection probes of the present invention. The 822 probe may be either the N822 wild-type probe or the N822K mutant probe, or both of them may be used in combination, for example.

When two or more kinds of polymorphism detection probe are used in combination, it is preferable that they are labeled probes having different labeling substances, for example. The different labeling substances may be, for example, labeling substances that are detected under different conditions.

In the present invention, the test nucleic acid may be a single-stranded nucleic acid or a double-stranded nucleic acid. When the test nucleic acid is a double-stranded nucleic acid, step (A) preferably includes the step of dissociating the double-stranded test nucleic acid by heating the reaction system, for example, as will be described below. By dissociating the double-stranded nucleic acid into single-stranded nucleic acids, the probe of the present invention can hybridize with the single-stranded nucleic acid, for example.

In the present invention, the test nucleic acid may be a nucleic acid contained inherently in a sample or an amplification product of the nucleic acid, for example. The latter is preferable because, for example, it allows the detection accuracy to be improved. The amplification product can be prepared by amplifying the nucleic acid in the sample as a template nucleic acid according to a nucleic acid amplification method, for example. The amplification product may be an amplification product obtained by using DNA in the sample as a template or an amplification product obtained by using cDNA synthesized from RNA in the sample as a template, for example. Examples of the RNA in the sample include RNAs such as total RNA and mRNA, and the cDNA can be synthesized from the RNA such as described above by RT-PCR (Reverse Transcription PCR), for example. The amplification product preferably is an amplification product of a region including the detection target sequence, for example. The detection target sequence may contain, as the detection target site, for example: only the detection target site of the 816 polymorphism; only the detection target site of the 822 polymorphism; or the detection target sites of both the 816 polymorphism and the 822 polymorphism.

For example, in the case where the test nucleic acid is the amplification product, the method of the present invention may further include, for example, the following step (X):
(X) producing the amplification product from a template nucleic acid.
The step (X) preferably is performed prior to step (A). Step (X) may be the step of producing the amplification product from the template nucleic acid in the reaction system in the presence of the polymorphism detection probe, for example.

In step (A), it is only necessary that the polymorphism detection probe is contained in the reaction sysytem, and the timing of the addition of the probe is not particulaly limited. In the case where the test nucleic acid is the amplification product, the reaction system in step (A) may be newly prepared using the amplification product obtained in step (X) and the probe, or may be the reaction system of the amplification reaction in step (X), for example. In the latter case, the probe may be added to the reaction system of the amplification reaction before or during step (X). Alternatively, the probe may be added to the reaction system after step (X).

The method for amplifying the nucleic acid is not particularly limited, and examples thereof include a PCR (Polymerase Chain Reaction) method, a NASBA (Nucleic Acid Sequence Based Amplification) method, a TMA (Transcription-Mediated Amplification) method, and a SDA (Strand Displacement Amplification) method. Among them, the PCR method is preferable. The conditions of the method for amplifying the nucleic acid are not particularly limited, and the method can be carried out using conventionally known techniques.

In the production of the amplification product from the template nucleic acid, it is preferable to use a primer for amplifying a region including a detection target polymorphism in the c-kit gene, for example. The sequence of the primer is not particularly limited as long as a detection target sequence including the detection target site can be amplified, for example, and the sequence of the primer can be set as appropriate by a conventionally known method, depending on the detection target sequence, sequences in the vicinity thereof, and the like. The length of the primer is not particularly limited, and can be set to a general length. For example, the length of the primer may be 10- to 50-mer, preferably 10- to 40-mer.

The region to be amplified with the primer is not particularly limited, and can be set as appropriate depending on a detection target polymorphism. When the detection target polymorphism is the 816 polymorphism, the region to be amplified preferably is a region including a detection target site of the 816 polymorphism, i.e., a region including the 108th base in the base sequence of SEQ ID NO: 1, for example. When the detection target polymorphism is the 822 polymorphism, the region to be amplified preferably is a region including a detection target site of the 822 polymorphism, i.e., a region including the 127th base in the base sequence of SEQ ID NO: 1, for example. When both the 816 polymorphism and 822 polymorphism are to be detected, the regions to be amplified may be, for example, two regions, namely, a region including the detection target site of the 816 polymorphism and a region including the detection target site of the 822 polymorphism, or a region including the detection target sites of both the 816 polymorphism and the 822 polymorphism, i.e., a region consisting of a sequence from the 108th to 127th bases in the base sequence of SEQ ID NO: 1 or a region including this sequence. When both the 816 polymorphism and the 822 polymorphism are to be detected, a region to be amplified preferably is a region including the sequence from the 108th to 127th bases in the base sequence of SEQ ID NO: 1 as described above, because this decreases the number of primers to be used, for example.

As the primer, for example, either one of a forward primer (hereinafter also referred to as "F primer") for amplifying the sense strand of the gene and a reverse primer (hereinafter also referred to as "R primer") for amplifying the antisense strand of the gene may be used. A primer set including a pair composed of these primers is preferable. Examples of the F primer and the R primer are shown below. It is to be noted, however, that they are merely illustrative and by no means limit the present invention.

Using a F primer composed of the following oligonucleotide F1 and an R primer composed of the following oligonucleotide R1 or R2, for example, it is possible to amplify a region including both the 816 polymorphism and the 822 polymorphism, i.e., a region including the sequence from the 108th to 127th bases in the base sequence of SEQ ID NO: 1.

### (F primer)

F1 (SEQ ID NO: 10)
   5'-tgtattcacagagacttggca-3'

### (R primer)

R1 (SEQ ID NO: 11)
   5'-gagaatgggtactcacgtttc-3'
R2 (SEQ ID NO: 12)
   5'-aaatcctttgcaggactgtc-3'

The F primer and the R primer each may be a primer designed so that it can anneal to a region including the detection target site, for example. In this case, the detection target site may be set to be either wild-type or mutant, for example. A primer that can anneal to a region including the wild-type detection target site can amplify a wild-type detection target sequence, for example, and also is referred to as a wild-type primer hereinafter. A primer that can anneal to a region including a mutant detection target site can amplify a mutant detection target sequence, for example, and also is referred to as a mutant primer hereinafter. In the primer, the position of the base to be paired with the detection target site is not particularly limited, and preferably is the 3' end, for example.

An example of a F primer that can anneal to a region including the detection target site of the 816 polymorphism will be given below. Such a primer is referred to as an 816 primer hereinafter. The primer is a primer for amplifying a c-kit gene, and is characterized in that it is composed of the following oligonucleotide (F2). In the following oligonucleotide, w is adenine (a) or thymine (t). According to this primer, for example, it is also possible to amplify a region including the detection target site of the 816 polymorphism and the detection target site of the 822 polymorphism.
(F2) a 10- to 50-mer oligonucleotide whose 3' end is the 108th base (w) in the base sequence of SEQ ID NO: 1

Examples of the oligonucleotide (F2) include the following oligonucleotides (F2-wt) and (F2-mt). The oligonucleotide (F2-wt) is configured so that the 3' end thereof can anneal to the D816 wild-type detection target site. The oligonucleotide (F2-mt) is configured so that the 3' end thereof can anneal to the D816V mutant detection target site.
(F2-wt) a 10- to 50-mer oligonucleotide whose 3' end is the 108th base (w) in the base sequence of SEQ ID NO: 1 and in which w is adenine (a)
(F2-mt) a 10- to 50-mer oligonucleotide whose 3' end is the 108th base (w) in the base sequence of SEQ ID NO: 1 and in which w is thymine (t)

The following (F2-wt1) is given as a specific example of (F2-wt), and the following (F2-mt1) is given as a specific example of (F2-mt). In each of the following sequences, the base indicated with a capital letter is a base that anneals to the detection target site.
F2-wt1 (SEQ ID NO: 13)
   5'-attttggtctagccagagA-3'
F2-mt1 (SEQ ID NO: 14)
   5'-tgattttggtctagccagagT-3'

An example of a F primer that can anneal to a region including the detection target site of the 822 polymorphism will be given below. Such a primer is referred to as an 822 primer hereinafter. The primer is a primer for amplifying the c-kit gene, and is characterized in that it is composed of the following oligonucleotide (F3). In the following oligonucleotide, d is thymine (t), guanine (g), or adenine (a).
(F3) a 10- to 50-mer oligonucleotide whose 3' end is the 127th base (d) in the base sequence of SEQ ID NO: 1

Examples of the oligonucleotide (F3) include the following oligonucleotides (F3-wt) and (F3-mt). The oligonucleotide (F3-wt) is configured so that the 3' end thereof can anneal to the N822 wild-type detection target site. The oligonucleotide (F3-mt) is configured so that d is r (guanine or adenine) and the 3' end thereof can anneal to the N822K mutant detection target site.
(F3-wt) a 10- to 50-mer oligonucleotide whose 3' end is the 127th base (d) in the base sequence of SEQ ID NO: 1 and in which d is thymine (t)
(F3-mt) a 10- to 50-mer oligonucleotide whose 3' end is the 127th base (d) in the base sequence of SEQ ID NO: 1 and in which d is r

The following (F3-wt1) is given as a specific example of (F3-wt), and the following (F3-mt1g) and (F3-mt1a) are given as specific examples of (F3-mt). In each of the following sequences, the base indicated with a capital letter is a base that anneals to the detection target site.
F3-wt1 (SEQ ID NO: 15)
   5'-cagagacatcaagaatgattctaaT-3'
F3-mt1g (SEQ ID NO: 16)
   5'-cagagacatcaagaatgattctaaG-3'
F3-mt1a (SEQ ID NO: 17)
   5'-cagagacatcaagaatgattctaaA-3'

Each of the F primer and the R primer may have an additional sequence added to its 5' end, for example. The length of the additional sequence is not particularly limited, and is, for example, 1- to 10-mer, preferably 2- to 7-mer. The additional sequence is not particularly limited, and examples thereof include sequences such as gatcg and tggca. An example of a F primer that has the additional sequence and can anneal to a region including the detection target site of the 822 polymorphism will be given below. F3-wt2 can anneal to a wild-type detection target site, and F3-mt2g and F3-mt2a can anneal to mutant detection target sites. In each of the following sequences, the additional sequence is underlined, and the base indicated with a capital letter is a base that anneals to the detection target site.
F3-wt2 (SEQ ID NO: 18)
   5'-gatcgcagagacatcaagaatgattctaaT-3'
F3-mt2g (SEQ ID NO: 19)
   5'-tggcacagagacatcaagaatgattctaaG-3'
F3-mt2a (SEQ ID NO: 20)
   5'-tggcacagagacatcaagaatgattctaaA-3'

In the present invention, the combination of the primers is not particularly limited. For example, it is preferable to use a pair composed of a F primer and a R primer as a primer set. Furthermore, it is preferable to use the wild-type primer and the mutant primer in combination as at least one of the F primer and the R primer. Specific examples of the combination include the combination of a F primer (wild-type F primer) for amplifying a wild-type detection target region, a F primer (mutant F primer) for amplifying a mutant detection target region, and a R primer. By using the wild-type primer and the mutant primer in combination, even in the case where a mutant c-kit gene is present in a trace amount, the mutant c-kit gene can be amplified more efficiently than a wild-type c-kit gene, for example.
As a result, the mutant detection target sequence can be detected with high accuracy and high sensitivity, for example.

The combination of the primer is not particularly limited, and examples thereof include the following combinations.
(1) combination of F2-wt1 with F2-mt1
(2) combination of F3-wt1 with F3-mt1g or F3-mt1a
(3) combination of F3-wt2 with F3-mt2g or F3-mt2a
(4) combination of at least any one of (1) to (3) with at least one of R1 and R2
(5) combination of F1 with at least one of R1 and R2

The combination of the primer and the probe is not particularly limited. Specifically, for example, it is preferable to use the following probe for the primer combinations (1) to (5) exemplified above.

The probe to be used for the primer combinations (1) and (4) is, for example, at least one of the 816 probe and the 822 probe, preferably the 816 probe, and more preferably the mutant 816 probe. The probe to be used for the primer combinations (2), (3), and (4) is, for example, the 822 probe, preferably the mutant 822 probe. The probe to be used for the primer combination (5) is, for example, at least one of the 816 probe and the 822 probe, preferably the 816 probe and the 822 probe, and more preferably the mutant 816 probe and the mutant 822 probe.

In the reaction system, the amount of primer to be added is not particularly limited. For example, the amount of one kind of primer to be added in the reaction system is, for example, 0.1 to 2 µmol/l, preferably 0.25 to 1.5 µmol/l, and particularly preferably 0.5 to 1 µmol/l. When a F primer and a R primer are used, the ratio (the molar ratio F : R) between the F primer (F) and R primer (R) to be added is not particularly limited, and preferably is 1 : 0.25 to 1 : 4, more preferably 1 : 0.5 to 1 : 2, for example.

In step (A), the ratio (molar ratio) of the probe of the present invention to be added relative to the test nucleic acid is not particularly limited. It preferably is 1 or less because this allows detection signals to be produced sufficiently. At this time, the amount of the test nucleic acid may be, for example, the total amount of a perfect match nucleic acid having a perfect match sequence and a mismatch nucleic acid having a mismatch sequence, or may be the total amount of an amplification product containing a perfect match sequence and an amplification product containing a mismatch sequence. Although the amount of perfect match nucleic acid in the test nucleic acid generally is unknown, it is preferable that the ratio (molar ratio) of the probe to be added relative to the perfect match nucleic acid (the amplification product containing the perfect match sequence) eventually becomes 10 or less, more preferably 5 or less, and still more preferably 3 or less. Furthermore, the lower limit of the ratio is not particularly limited, and is, for example, 0.001 or more, preferably 0.01 or more, and more preferably 0.1 or more. The ratio of the probe of the present invention to be added relative to the test nucleic acid may be the molar ratio thereof relative to a double-stranded nucleic acid or relative to a single-stranded nucleic acid, for example.

The amount of probe of the present invention to be added in the reaction system is not particularly limited. For example, it is preferable to add each kind of probe so that its concentration is in the range from 10 to 1000 nmol/l, more preferably from 20 to 500 nmol/l. Furthermore, in the reaction system, the molar ratio of the probe relative to the test nucleic acid preferably is, for example, 1 or less, because this allows sufficient signal values to be produced, for example. The ratio of the probe to be added relative to the test nucleic acid may be the molar ratio thereof relative to a double-stranded nucleic acid or relative to a single-stranded nucleic acid, for example.

A sample to which the polymorphism detection method of the present invention is applied is not particularly limited, and examples thereof include biological samples. Specific examples of the biological samples include: whole blood; blood cells such as leukocyte cells; bone marrow; oral cells such as oral mucosa; somatic cells such as nail and hair cells; germ cells; sputum; amniotic fluid; paraffin-embedded tissues; urine; gastric juice; and liquid obtained by gastrolavage. In the present invention, a method for collecting the sample, a method for preparing a test nucleic acid from the sample, and the like are not particularly limited, and any conventionally known methods can be employed.

The polymorphism detection method of the present invention can be utilized in so-called Tm analysis (also referred to as melting curve analysis) such as described above. The following is an explanation of a Tm value in Tm analysis. For example, when a solution containing a double-stranded DNA is heated, absorbance at 260 nm increases. This is caused by the fact that the hydrogen bonds between the strands making up the double-stranded DNA are broken by the heating, whereby the double-stranded DNA is dissociated into single-stranded DNAs (melting of DNA). Then, when every double-stranded DNA is dissociated into single-stranded DNAs, the absorbance of the solution becomes about 1.5 times as large as the absorbance at the time when the heating was initiated (i.e., the absorbance of the solution containing only the double-stranded nucleic acid), whereby it can be determined that the melting is complete. Based on this phenomenon, the melting temperature Tm generally is defined as the temperature at the time when the amount of increase in absorbance reaches 50% of the total amount of increase in absorbance.

In step (A), the measurement of a signal indicating the melting state of a hybrid of the test nucleic acid and the probe may be the measurement of absorbance at 260 nm as described above or the measurement of a signal of the labeling substance. Specifically, it is preferable that a labeled probe labeled with the labeling substance is used as the probe as described above and that a signal of the labeling substance is measured. The labeled probe may be, for example, a labeled probe that shows a signal independently and shows no signal when it forms a hybrid, or a labeled probe that shows no signal independently and shows a signal when it forms a hybrid. The former probe does not show a signal when it forms a hybrid (double-stranded DNA) with the amplification product and shows a signal when the probe is dissociated from the amplification product by heating. On the other hand, the latter probe shows a signal when it forms a hybrid (double-stranded DNA) with the amplification product, and the signal is reduced (quenched) when the probe is dissociated from the amplification product by heating. Therefore, by detecting a signal of the labeling substance, detection of the progress of the melting of the hybrid, determination of the Tm value, and the like can be achieved, as in the case where absorbance at 260 nm is measured. The signal of the labeling substance may be detected under conditions specific to the signal of the labeling substance, for example. Examples of these conditions include an excitation wavelength and a detection wavelength. The labeled probe and the labeling substance are as described above.

In step (B), detection of the polymorphism based on the change in signal value can be carried out by a conventional method. Specifically, for example, by comparing the above-described change in signal value with the same regarding a hybrid of the probe and a mutant detection target sequence and/or a hybrid of the probe and a wild-type detection target sequence, it is possible to determine whether the polymorphism is mutant or wild-type. That is, the polymorphism can be determined as mutant when the change in signal value is the same as that of the hybrid with the mutant target sequence, whereas the polymorphism can be determined as wild-type when the change in signal value is the same as that of the hybrid with the wild-type target sequence. Alternatively, for example, the polymorphism can be determined by determining the Tm value based on the change in signal and then comparing the thus-determined Tm value. First, the Tm value is determined based on the change in signal value. Then, the measured Tm value is compared with a previously determined evaluation standard, i.e., a Tm_{wt} value regarding the wild-type detection target sequence and/or a Tmₘₜ value regarding the mutant detection target sequence. The polymorphism can be determined as: wild-type when the measured Tm value is the same as or similar to the Tm_{wt} value as the evaluation standard; mutant when the measured Tm value is lower than the Tm_{wt} value; mutant when the measured Tm value is the same as or similar to the Tmₘₜ value as the evaluation standard; and wild-type when the measured Tm value is lower than the Tmₘₜ value. Note here that the values "similar" to each other as used herein means values with difference of about ±3°C, for example.

Next, the polymorphism detection method of the present invention will be described with reference to an illustrative example. The present example is directed to the case where the probe of the present invention is a labeled probe labeled with a fluorescent substance, a template nucleic acid is amplified in the presence of the probe, and the resultant amplification product is used as the test nucleic acid. The polymorphism detection method of the present invention is characterized in that the probe of the present invention is used in the method, and other steps and conditions are by no means limited.

First, genomic DNA is isolated from the biological sample. Isolation of the genomic DNA from the biological sample can be achieved by a conventionally known method. Specifically, the isolation can be achieved using a commercially available genomic DNA isolation kit (GFX Genomic Blood DNA Purification kit™; GE Healthcare Bio-Sciences) or the like, for example.

Next, a reaction solution is prepared by adding a labeled probe to a sample containing the isolated genomic DNA. As the labeled probe, for example, QProbe® (registered trademark) is preferable, as described above.

The labeled probe may be added to the sample containing the isolated genomic DNA or may be mixed with the genomic DNA in a solvent, for example. The solvent is not particularly limited, and examples thereof include conventionally known solvents including: buffer solutions such as Tris-HCl; solvents respectively containing KCl, MgCl₂, MgSO₄, glycerol, and the like; and reaction solutions for amplification, such as reaction solutions for PCR.

The timing of the addition of the labeled probe is not particularly limited. For example, the labeled probe may be added before, during, or after the amplification reaction. In particular, it is preferable to add the labeled probe to the reaction solution before the amplification reaction because it is not necessary to expose the reaction solution to the external environment in order to add the labeled probe and it is possible to carry out the amplification reaction and the measurement of signal values successively, for example. In this case, it is preferable that the 3' end of the labeled probe is modified with the labeling substance or a phosphate group, as described above.

Subsequently, using the isolated genomic DNA as a template, a sequence including a detection target site at which a detection target polymorphism occurs is amplified in the presence of the labeled probe by a nucleic acid amplification method such as PCR. Although the present invention will be described below with reference to the case where PCR is used as the nucleic acid amplification method, the present invention is not limited thereto. Also, conditions for the PCR are not particularly limited, and the PCR can be carried out according to a conventionally known method.

Specifically, PCR is carried out with respect to the reaction solution containing the genomic DNA, the labeled probe, and the primer. The composition of this reaction solution is not particularly limited, and those skilled in the art can set the composition as appropriate. In addition to the genomic DNA, the labeled probe, and the primer, the reaction solution further may contain: a polymerase such as DNA polymerase; nucleoside triphosphate; a buffer solution; any of various kinds of catalysts; and the like, for example. The amount of the labeled probe and the primer to be added in the reaction solution is not particularly limited, and may be in the above-described ranges, respectively, for example.

The DNA polymerase is not particularly limited, and conventionally known polymerases derived from heat-resistant bacteria can be used, for example. As specific examples of such polymerases, *Thermus* aquaticus-derived DNA polymerases (U.S. Patent Nos. 4,889,818 and 5,079,352) (Taqpolymerase™), *Thermus thermophilus-derived* DNA polymerase (WO 91/09950) (rTth DNA polymerase), *Pyrococcus* furiosus-derived DNA polymerase (WO 92/9689) (Pfu DNA polymerase: Stratagene), *Thermococcus litoralis-derived* polymerase (EP-A 455 430 (Vent™): New England Biolabs), and the like, for example, are commercially available. Among them, *Thermus aquaticus*-derived heat-resistant DNA polymerases are preferable.

The amount of DNA polymerase to be added in the reaction solution is not particularly limited, and is, for example, 1 to 100 U/ml, preferably 5 to 50 U/ml, and more preferably 20 to 40 U/ml. With regard to the unit of activity (U) of DNA polymerases, 1 U generally is defined as the activity for incorporating 10 nmol of total nucleotides into an acid-insoluble precipitate at 74°C in 30 minutes in a reaction solution for activity measurement using activated salmon sperm DNA as a template primer. The composition of the reaction solution for activity measurement is as follows, for example: 25 mmol/l TAPS buffer (pH 9.3, 25°C), 50 mmol/l KCl, 2 mmol/l MgCl₂, 1 mmol/l mercaptoethanol, 200 µmol/l dATP, 200 µmol/l dGTP, 200 µmol/l dTTP, 100 µmol/l [α-³²P] dCTP, and 0.25 mg/ml activated salmon sperm DNA.

The nucleoside triphosphate generally is dNTP (dATP, dCTP, dGTP, and dTTP or dUTP). The amount of dNTP to be added in the reaction solution is not particularly limited, and is, for example, 0.01 to 1 mmol/l, preferably 0.05 to 0.5 mmol/l, and more preferably 0.1 to 0.3 mmol/l.

Examples of the buffer solution include Tris-HCl, Tricine, MES, MOPS, HEPES, and CAPS, and it is possible to use commercially available buffer solutions for PCR and buffer solutions included in commercially available PCR kits.

The reaction solution further may contain heparin, betaine, KCl, MgCl₂, MgSO₄, glycerol, or the like, and the amount of these components to be added may be set within ranges where they do not interfere with the PCR reaction.

The total volume of the reaction solution is not particularly limited, and can be determined as appropriate depending on the device to be used, such as a thermal cycler, and the like, for example. The total volume generally is 1 to 500 µl, preferably 10 to 100 µl.

Next, PCR is conducted. The cycle conditions of the PCR are not particularly limited. For example, conditions shown in Table 1 below can be employed, respectively, for: (1) dissociation of a double-stranded DNA as the test nucleic acid into single-stranded DNAs; (2) annealing of the primer to the single-stranded DNA; and (3) elongation of the primer through a polymerase reaction. The number of cycles is not particularly limited. It preferably is 30 cycles or more with the three steps described in the following items (1) to (3) as one cycle, for example. The upper limit of the total number of cycles is not particularly limited, and is, for example, 100 cycles or less, preferably 70 cycles or less, and more preferably 50 cycles or less. The temperature change in each of the steps can be controlled automatically using a thermal cycler or the like, for example.

**[Table 1]**

| | Temperature (°C) and Time (seconds) |
|---|---|
| (1) Dissociation into single-stranded DNAs | e.g., 90°C, 1 to 120 seconds |
| | preferably, 92°C to 95°C, 1 to 60 seconds |
| (2) Annealing of primer | e.g., 40°C to 70°C, 1 to 300 seconds |
| | preferably, 50°C to 70°C, 5 to 60 seconds |
| (3) Elongation of primer | e.g., 50°C to 80°C, 1 to 300 seconds |
| | preferably, 50°C to 75°C, 5 to 60 seconds |

The amount of the labeled probe to be added in the reaction solution is not particularly limited. For example, it is preferable to add the labeled probe so that its concentration is in the range from 10 to 1000 nmol/l, more preferably from 20 to 500 nmol/l. In the reaction solution, the molar ratio of the labeled probe relative to the test nucleic acid preferably is, for example, 1 or less, because this allows sufficient signal to be produced, for example.
The ratio of the labeled probe to be added relative to the test nucleic acid may be the molar ratio thereof relative to a double-stranded nucleic acid or relative to a single-stranded nucleic acid, for example.

Next, dissociation of the obtained amplification product (double-stranded DNA) and hybridization of the labeled probe with a single-stranded DNA obtained through the dissociation are carried out.
They can be achieved by, for example, changing the temperature of the reaction solution in the presence of the labeled probe. In this case, it is preferable that the reaction solution to which the labeled probe has been added is subjected to an amplification reaction, after which the temperature of the reaction solution is changed, as described above.

The heating temperature in the dissociation step is not particularly limited as long as it is a temperature at which the double-stranded amplification product can be dissociated into single strands. For example, the heating temperature is 85°C to 95°C. The heating time is not particularly limited, and generally is 1 second to 10 minutes, preferably 1 second to 5 minutes.

The hybridization of the labeled probe with the dissociated single-stranded DNA can be achieved by, for example, lowering the heating temperature in the dissociation step after completion of the dissociation step. The temperature condition is 40°C to 50°C, for example. The time period for conducting the treatment at this temperature is not particularly limited, and is, for example, 1 to 600 seconds.

Then, signal values indicating the melting states of the hybrid of the amplification product and the labeled probe are measured while changing the temperature of the reaction solution. Specifically, for example, the reaction solution (the hybrid of the single-stranded DNA and the labeled probe) is heated, and the change in signal value accompanying the temperature rise is measured. As described above, in the case where a guanine quenching probe, i.e., a probe in which cytosine (c) at the end is labeled, is used, fluorescence is reduced (or quenched) when the probe hybridizes with the single-stranded DNA, and fluorescence is emitted when the probe is dissociated. Therefore, in this case, the hybrid with reduced (quenched) fluorescence may be heated gradually, and the increase in fluorescence intensity accompanying the temperature rise may be measured, for example.

The temperature range when measuring the change in fluorescence intensity is not particularly limited. The initiation temperature is, for example, room temperature to 85°C, preferably 25°C to 70°C, and the end temperature is, for example, 40°C to 105°C. The temperature rising rate is not particularly limited, and is, for example, 0.1 to 20 °C/sec., preferably 0.3 to 5 °C/sec.

Next, the Tm value is determined by analyzing the change in signal value. Specifically, from the obtained fluorescence intensities, the amount of change in fluorescence intensity per unit time (-d amount of change in fluorescence intensity/dt) at each temperature is calculated, and the lowest temperature can be determined as the Tm value. Alternatively, the temperature at which the amount of change in fluorescence intensity per unit time (d amount of increase in fluorescence intensity/dt) is largest can be determined as the Tm value. When a probe that shows no signal independently and shows a signal when it forms a hybrid is used as the labeled probe instead of the fluorescence quenching probe, the amount of decrease in fluorescence intensity may be measured, contrary to the case stated above. Alternatively, for example, a plurality of probes that are respectively labeled with labeling substances to be detected at different detection wavelengths may be used as the labeled probes, and the change in signal value may be analyzed at each detection wavelength.

The Tm value can be calculated using conventionally known MELTCALC software (http://www.meltcalc.com/) or the like, for example. Also, the Tm value can be determined by a nearest neighbour method.

Then, based on the Tm value, whether the polymorphism in the detection target sequence of the c-kit gene is wild-type or mutant is determined. In Tm analysis, a perfectly complementary hybrid (match) exhibits a higher Tm value indicating dissociation than a hybrid with one or more different bases (mismatch). Therefore, regarding the labeled probe, by determining the Tm value of a hybrid that shows a match with the detection target site (e.g., a perfectly complementary hybrid) and the Tm value of a hybrid that shows a mismatch with the detection target site (e.g., a hybrid with one or more different bases) beforehand as evaluation standard values, it is possible to determine whether the polymorphism in the detection target sequence is wild-type or mutant. As described above, by using a wild-type probe and a mutant probe in combination, the kind of polymorphism also can be determined depending on which of the probes shows the Tm value of the perfectly complementary hybrid, for example.

For example, when the mutant probe is used as the probe, it can be determined that the polymorphism is mutant when the probe shows the Tm value of the perfectly complementary match hybrid and that the polymorphism is wild-type when the probe shows the Tm value of the mismatch hybrid. On the other hand, for example, when the wild-type probe is used as the probe, it can be determined that the polymorphism is wild-type when the probe shows the Tm value of the perfectly complementary match hybrid and that the polymorphism is mutant when the probe shows the Tm value of the mismatch hybrid.

In the present invention, instead of raising the temperature of the reaction system containing the probe (i.e., heating the hybrid), and then measuring the change in the signal accompanying the temperature rise as described above, the change in the signal at the time of hybrid formation may be measured, for example. That is, when a hybrid is formed by lowering the temperature of the reaction system containing the probe, the change in signal accompanying the lowering of the temperature may be measured.

Specifically, in the case where a labeled probe that shows a signal independently and shows no signal when it forms a hybrid (e.g., a guanine quenching probe) is used, the labeled probe emits fluorescence when a single-stranded DNA and the labeled probe are dissociated, and the fluorescence is reduced (or quenched) when the temperature is lowered to allow the labeled probe to form a hybrid. Therefore, in this case, the temperature of the reaction solution may be lowered gradually, and the decrease in fluorescence intensity accompanying the temperature lowering may be measured, for example. On the other hand, when a labeled probe that shows no signal independently and shows a signal when it forms a hybrid is used, the labeled probe does not emit fluorescence when the single-stranded DNA and the labeled probe are dissociated, and the labeled probe emits fluorescence when the temperature is lowered to allow the labeled probe to form a hybrid. Therefore, in this case, the temperature of the reaction solution may be lowered gradually, and the increase in fluorescence intensity accompanying the lowering of the temperature may be measured, for example.

In the present invention, as described above, two or more kinds of probes can be used in a single reaction system, for example. When a plurality of probes is used, the timing of the addition of the respective probes is as described above, for example, and they preferably are added at the same time prior to step (A). When an amplification reaction is to be carried out, they preferably are added at the same time prior to the amplification reaction.

The plurality of probes may be the combination of a D816 wild-type probe and a D816V mutant probe, or the combination of an N822 wild-type probe and an N822K mutant probe, for example. Also, the plurality of probes may be, for example, the combination of an 816 polymorphism probe and an 822 polymorphism probe, and examples of this combination include the combination of at least one of a D816 wild-type probe and a D816V mutant probe with at least one of an N822 wild-type probe and an N822K mutant probe.

When both the 816 polymorphism and the 822 polymorphism are to be detected in a single reaction system, the 816 probe and the 822 probe may be used in combination, for example. At this time, the 816 probe and the 822 probe preferably have different labeling substances, as described above. The Tm values of the respective probes can be determined by changing the temperature of the reaction solution and detecting a signal under measurement conditions appropriate for the respective labeling substances.

Also, as described above, a wild-type probe and a mutant probe may be used in combination for the detection of a target polymorphism. In this case, the kind of polymorphism also can be determined depending on which of the probes shows the Tm value of the perfectly complementary (match) hybrid, for example. For example, in the case where a mutant probe and a wild-type probe having different labeling substances are used, it can be determined that the polymorphism is mutant when the mutant probe shows the Tm value of the perfectly complementary (match) hybrid, and that the polymorphism is wild-type when the wild-type probe shows the Tm value of the perfectly complementary (match) hybrid.

In the polymorphism detection method of the present invention, for example, the probe for detecting a polymorphism in a c-kit gene according to the present invention can be used in combination with a probe for detecting a polymorphism in any other gene. By using the probe of the present invention in combination with a probe for detecting any other gene, polymorphisms of two or more kinds of genes including the c-kit gene can be detected in the same single reaction system.

### <Probe Reagent>

The probe reagent according to the present invention is characterized in that it contains the polymorphism detection probe of the present invention. The probe of the present invention is as described above, and the probe reagent may contain one kind of the probe or two or more kinds of the probes. Examples of the combination of probes include, but are not limited to, those described above.

### <Primer and Primer Reagent>

The primer of the present invention is a primer for amplifying a c-kit gene, characterized in that it is composed of either one of the following oligonucleotides (F2) and (F3).
(F2) a 10- to 50-mer oligonucleotide whose 3' end is the 108th base w in the base sequence of SEQ ID NO: 1
(F3) a 10- to 50-mer oligonucleotide whose 3' end is the 127th base d in the base sequence of SEQ ID NO: 1

The primer composed of the oligonucleotide (F2) is the 816 primer, and w is adenine (a) or thymine (t). The primer composed of the oligonucleotide (F3) is the 822 primer, and d is thymine (t), guanine (g), or adenine (a). The oligonucleotides (F2) and (F3) are as described above.

The primer reagent of the present invention is a reagent containing a primer for amplifying a c-kit gene, and is characterized in that it contains the primer of the present invention. The primer reagent of the present invention may be any reagent as long as it contains the primer of the present invention, and other configurations are by no means limited. The primer reagent of the present invention may further contain a reverse primer for amplifying a sense strand.

The primer reagent of the present invention may contain one kind of primer of the present invention or two or more kinds of primers of the present invention. The kind of primer can be determined as appropriate depending on the target region to be amplified, for example. When a detection target sequence including the 816 polymorphism is to be amplified, it is preferable that the primer reagent contains the oligonucleotide (F2), and it is particularly preferable that the primer reagent contains the oligonucleotides (F2-wt) and (F2-mt). When a detection target sequence including the 822 polymorphism is to be amplified, it is preferable that the primer reagent contains the oligonucleotide (F3), and it is particularly preferable that the primer reagent contains the oligonucleotides (F3-wt) and (F3-mt). When the primer reagent contains both a wild-type primer and a mutant primer as primers that can anneal to detection target sites, it is possible to specifically amplify a trace amount of a mutant detection target sequence, for example. Examples of the combination of the primers include, but are not limited to, those described above.

### <Polymorphism Detection Reagent>

The polymorphism detection reagent of the present invention is a reagent for detecting a polymorphism in a c-kit gene, containing the polymorphism detection probe of the present invention. The polymorphism detection reagent of the present invention is characterized in that it contains the above-described polymorphism detection probe of the present invention, and other configurations and conditions are by no means limited. The polymorphism detection reagent of the present invention also can be referred to as a probe kit for use in the detection of a polymorphism in a c-kit gene, for example.

The polymorphism detection reagent may contain one kind of the probe or two or more kinds of the probes. The number of kinds of probe to be contained in the polymorphism detection reagent can be determined as appropriate depending on the detection target polymorphism(s), for example. In the case where only the 816 polymorphism is to be detected, the polymorphism detection reagent preferably contains the 816 probe, for example. The 816 probe may be either the D816 wild-type probe or the D816V mutant probe, or the polymorphism detection reagent may contain both of them, for example. In the case where only the 822 polymorphism is to be detected, the polymorphism detection reagent preferably contains the 822 probe, for example. The 822 probe may be either the N822 wild-type probe or the N822K mutant probe, or the polymorphism detection reagent may contain both of them, for example. Examples of the combination of probes include, but are not limited to, those described above.

The polymorphism detection reagent of the present invention further may contain a primer or primer set for amplifying a region including a detection target site in a c-kit gene. Examples of the primer include those described above. Preferably, the polymorphism detection reagent contains the primer of the present invention. Examples of the combination of primers include, but are not limited to, those described above.

The polymorphism detection reagent of the present invention further may contain components necessary for a nucleic acid amplification reaction, for example. Specific examples of such components include: polymerases such as DNA polymerases; nucleoside triphosphate; buffer solutions; and various kinds of catalysts. The polymorphism detection reagent of the present invention may be provided as a detection reagent kit, and may further include instructions for use.

Next, examples of the present invention will be described. It is to be noted, however, the present invention is by no means limited by the following examples. In the following examples, "%" means "w/v%" regarding BSA and NaN3, "v/v%" regarding glycerol, and "w/w%" regarding other components.

### Examples

### [Example 1]

In the present example, Tm analysis was carried out in the presence of a wild-type plasmid and mutant plasmids to detect polymorphisms in a c-kit gene.

A wild-type plasmid (WT) obtained through insertion of an oligonucleotide having D816 wild-type and N822 wild-type (SEQ ID NO: 23) as a partial sequence of the c-kit gene, a D816V mutant plasmid (D816V) obtained through insertion of an oligonucleotide having the D816V mutant and the N822 wild-type (SEQ ID NO: 24) as a partial sequence of the c-kit gene, and an N822K mutant plasmid (N822K) obtained through insertion of an oligonucleotide having the D816 wild-type and the N822K mutant (SEQ ID NO: 25) as a partial sequence of the c-kit gene were provided. In the wild-type plasmid (WT), the 108th base (w) and the 127th base (d) in the base sequence of SEQ ID NO: 1 are adenine (a) and thymine (t), respectively. In the D816V mutant plasmid (D816V), the 108th base (w) and the 127th base (d) in the base sequence of SEQ ID NO: 1 are thymine (t) and thymine (t), respectively. In the N822K mutant plasmid (N822K), the 108th base (w) and the 127th base (d) in the base sequence of SEQ ID NO: 1 are adenine (a) and guanine (g), respectively. In SEQ ID NOs: 23 to 25 shown below, underlined bases indicated with capital letters correspond to the 108th and 127th bases in the base sequence of SEQ ID NO: 1.

WT (SEQ ID NO: 23)
5'-cactatagtattaaaaagttagttttcactctttacaagttaaaatgaatttaaatggttttcttttctcctcca
acctaatagtgtattcacagagacttggcagccagaaatatcctccttactcatggtcggatcacaaagatttgt
gattttggtctagccagagAcatcaagaatgattctaaTtatgtggttaaaggaaacgtgagtacccattctc
tgcttgacagtcctgcaaaggatttttagtttcaactttcgataaaaattgtttcctgtgactttcataatgtaaa
t-3'

D816V (SEQ ID NO: 24)
5'-cactatagtattaaaaagttagttttcactctttacaagttaaaatgaatttaaatggttttcttttctcctcca
acctaatagtgtattcacagagacttggcagccagaaatatcctccttactcatggtcggatcacaaagatttgt
gattttggtctagccagagTcatcaagaatgattctaaTtatgtggttaaaggaaacgtgagtacccattctc
tgcttgacagtcctgcaaaggatttttagtttcaactttcgataaaaattgtttcctgtgactttcataatgtaaa
t-3'

N822K (SEQ ID NO: 25)
5'-cactatagtattaaaaagttagttttcactctttacaagttaaaatgaatttaaatggttttcttttctcctcca
acctaatagtgtattcacagagacttggcagccagaaatatcctccttactcatggtcggatcacaaagatttgt
gattttggtctagccagagAcatcaagaatgattctaaGtatgtggttaaaggaaacgtgagtacccattctc
tgcttgacagtcctgcaaaggatttttagtttcaactttcgataaaaattgtttcctgtgactttcataatgtaaa
t-3'

These plasmids were mixed together at the predetermined proportion shown in the following table. Thus, three kinds of plasmid samples were prepared. The plasmid content in each plasmid sample was set to 2 x 10⁴ copies/µl.

**[Table 2]**

| (Plasmid Sample) Plasmid sample | Mixed ratio of respective plasmids | | |
|---|---|---|---|
| | WT | D816V | N822K |
| WT 100% | 100% | 0% | 0% |
| D816V 20% | 80% | 20% | 0% |
| N822K 20% | 80% | 0% | 20% |

50 µl of the PCR reaction solution shown in Table 3 below was subjected to PCR and Tm analysis using a fully-automated SNP analyzer (i-densy®, ARRAY, Inc.). The PCR was carried out in the following manner: the PCR reaction solution was first treated at 95°C for 60 seconds, and then was subjected to 50 cycles of treatment with a treatment at 95°C for 1 second and at 58°C for 15 seconds as one cycle. Subsequently, Tm analysis was carried out by treating the reaction solution at 95°C for 1 second and 40°C for 60 seconds, then heating the reaction solution from 40°C to 95°C at a temperature rising rate of 1°C/3 seconds, and measuring the change in fluorescence intensity with time at detection wavelengths 445 to 480 nm (for Pacific Blue) and 520 to 555 nm (for BODIPY FL).

**[Table 3]**

| (Composition of PCR reaction solution: unit µl) | |
|---|---|
| distilled water | 35.135 |
| 1 mol/l Tris-HCl (pH 8.6) | 1.25 |
| 20% BSA | 0.5 |
| 10% NaN₃ | 0.23 |
| 80% glycerol | 1.56 |
| 1 mol/l MgCl₂ | 0.075 |
| 1 mol/l KCl | 1.25 |
| 2.5 mmol/l dNTP | 4 |
| 100 µmol/l F primer | 0.5 |
| 100 µmol/l R primer | 0.25 |
| 5 µmol/l 816 probe | 2 |
| 5 µmol/l 822 probe | 2 |
| 5 U/µl Gene Taq (NIPPON GENE) | 0.25 |
| Plasmid sample | 1 |
| Total | 50 µl |

The sequences of the F primer and R primer are shown below.

### (F primer)

F1 (SEQ ID NO: 10)
   5'-tgtattcacagagacttggca-3'

### (R primer)

R1 (SEQ ID NO: 11)
   5'-gagaatgggtactcacgtttc-3'

As the 816 probe and the 822 probe, probes with the following sequences were used, respectively. The following D816V mutant probe 1 is a probe that shows a perfect match with a detection target sequence in the sense strand of the D816V mutant c-kit gene. In the sequence, the underlined base is complementary to the D816V mutant. The 3' end of the D816V mutant probe 1 was labeled with a fluorescent dye "BODIPY FL". The following N822K mutant probe 1 is a probe that shows a perfect match with a detection target sequence in the sense strand of the N822K mutant c-kit gene. In the sequence, the underlined base is complementary to the N822K mutant. The 5' end of the N822K mutant probe 1 was labeled with a fluorescent dye "Pacific Blue", and the 3' end of the N822K mutant probe 1 was phosphorylated.

### (816 probe)

D816V mutant probe 1 (SEQ ID NO: 6)
   5'-aatcattcttgatgactc-(BODIPY FL)-3

### (822 probe)

N822K mutant probe 1 (SEQ ID NO: 8)
   5'-(Pacific Blue)-ccacatacttagaatcattctt-P-3

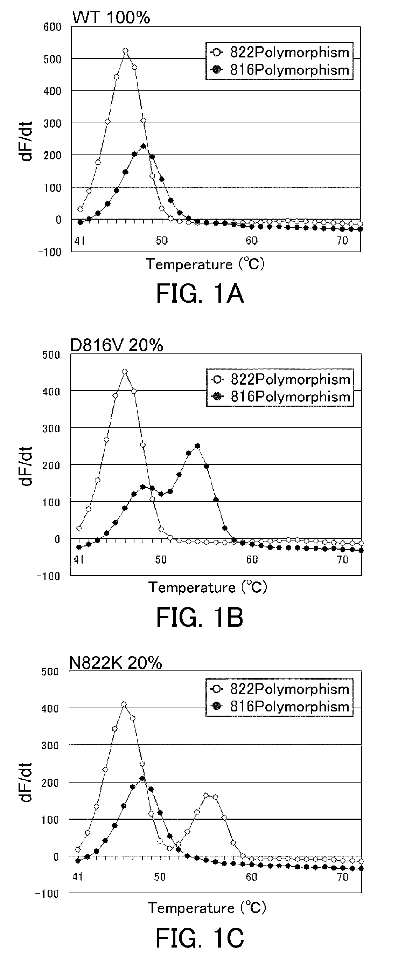

The results are shown in FIG. 1. FIG. 1 shows graphs each showing the results of Tm analysis, indicating the change in fluorescence intensity accompanying the temperature rise. FIG. 1A shows the result obtained regarding the plasmid sample WT 100%, FIG. 1B shows the result obtained regarding the plasmid sample D816V 20%, and FIG. 1C shows the result obtained regarding the plasmid sample N822K 20%. In FIG. 1, the plot indicated with open circles (o) is the melting curve regarding the 822 probe, and the plot indicated with filled circles (●) is the melting curve regarding the 816 probe. The horizontal axis indicates the temperature (°C) at the time of measurement. The vertical axis indicates the change in fluorescence intensity (hereinafter also referred to as the "amount of change in fluorescence"), and the unit thereof is "d amount of increase in fluorescence intensity/dt" (dF/dt), which indicates the differential value of the amount of change in fluorescence intensity. The Tm value of a hybrid of the D816V mutant probe 1 with D816 (WT) is around 48°C, and the Tm value of a hybrid of the D816V mutant probe 1 with D816V is around 54°C. The Tm value of a hybrid of the N822K mutant probe 1 with N822 (WT) is around 46°C, and the Tm value of a hybrid of the N822K mutant probe 1 with N822K is around 55°C.

As can be seen from FIG. 1A, when the plasmid sample WT 100% was used, peaks were observed only at the Tm value of D816 and at the Tm value of N822. On the other hand, when the plasmid samples containing both the wild-type plasmid and the mutant plasmid were used, peaks were observed at two Tm values regarding the polymorphism of the mutant plasmid contained therein. More specifically, as can be seen from FIG. 1B, when the plasmid sample D816V 20% was used, peaks were observed at both the Tm values of D816 and D816V and at the Tm value of N822. As can be seen from FIG. 1C, when the plasmid sample N822K 20% was used, peaks were observed at the Tm value of D816 and at both the Tm values of N822 and N822K. These results demonstrate that, by using the wild-type F primer, wild-type R primer, and mutant probe for each polymorphism according to the present example, even in the case where a wild-type polymorphism and a mutant-type polymorphism are present together, it is possible to discriminately detect the wild-type polymorphism and the mutant polymorphism and also to detect two kinds of polymorphisms in the same single reaction system.

### [Example 2]

In the present example, Tm analysis was carried out in the presence of a wild-type plasmid and a mutant plasmid to detect the 816 polymorphism in a c-kit gene.

The wild-type plasmid (WT) and the D816V mutant plasmid (D816V) obtained through insertion of the oligonucleotide having D816V mutant used in Example 1 were mixed together at the predetermined proportion shown in the following table. Thus, three kinds of plasmid samples were prepared. The plasmid content in each plasmid sample was set to 2 x 10⁴ copies/µl.

**[Table 4]**

| (Plasmid Sample) Plasmid sample | Mixed ratio of respective plasmids | |
|---|---|---|
| | WT | D816V |
| WT 100% | 100% | 0% |
| D816V 0.3% | 99.7% | 0.3% |
| D816V 1% | 99% | 1% |

Using 50 µl of a PCR reaction solution shown in Table 5 below, PCR and Tm analysis were conducted in the same manner as in Example 1, except that conditions for the PCR and Tm analysis were set as follows. The PCR and Tm analysis were conducted in the following manner. The PCR reaction solution was first treated at 95°C for 60 seconds, then was subjected to 50 cycles of treatment with a treatment at 95°C for 1 second and at 61°C for 15 seconds as one cycle, and further was treated at 95°C for 1 second and 40°C for 60 seconds. Subsequently, the PCR reaction solution was heated from 40°C to 75°C at a temperature rising rate of 1°C/3 seconds. The detection wavelength was set to 585 to 700 nm (for TAMRA).

**[Table 5]**

| (Composition of PCR reaction solution: unit µl) | | |
|---|---|---|
| distilled water | | 37.135 |
| 1 mol/l Tris-HCl (pH 8.6) | | 1.25 |
| 20% BSA | | 0.5 |
| 10% NaN₃ | | 0.23 |
| 80% glycerol | | 1.56 |
| 1 mol/l MgCl₂ | | 0.075 |
| 1 mol/l KCl | | 1.25 |
| 2.5 mmol/l dNTP | | 4 |
| 100 µmol/l wild-type F primer | | 0.125 |
| 100 µmol/l mutant F primer | | 0.125 |
| 100 µmol/l R primer | | 0.5 |
| 5 µmol/l probe | | 2 |
| 5 U/µl Gene Taq (NIPPON GENE) | | 0.25 |
| Plasmid sample | | 1 |
| | Total | 50 µl |

The sequences of the wild-type F primer, mutant F primer, and R primer are shown below.

### (wild-type F primer)

F2-wt1 (SEQ ID NO: 13)
   5'-attttggtctagccagaga-3'

### (mutant F primer)

F2-mt1 (SEQ ID NO: 14)
   5'-tgattttggtctagccagagt-3'

### (R primer)

R2 (SEQ ID NO: 12)
   5'-aaatcctttgcaggactgtc-3'

As the probe, the following D816V mutant probe 2 was used. The probe shows a perfect match with a detection target sequence in the antisense strand of the D816V mutant c-kit gene. In the sequence, the underlined base is the base complementary to the D816V mutant. The 5' end of the D816V mutant probe 2 was labeled with a fluorescent dye "TAMRA", and the 3' end of the D816V mutant probe 2 was phosphorylated.
5'-(TAMRA)-ccagagtcatcaagaatg-P-3' (SEQ ID NO: 7)

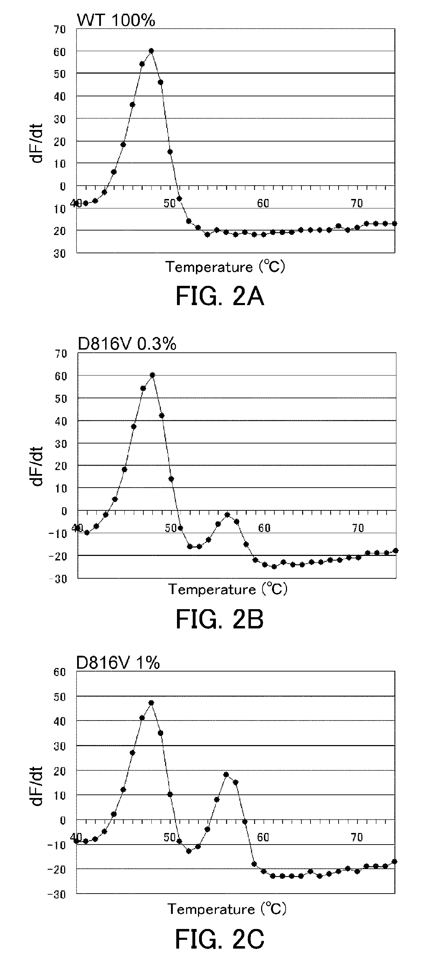

The results are shown in FIG. 2. FIG. 2 shows graphs each showing the result of Tm analysis, indicating the change in fluorescence intensity accompanying the temperature rise. FIG. 2A shows the result obtained regarding the plasmid sample WT 100%; FIG. 2B shows the result obtained regarding the plasmid sample D816V 0.3%; and FIG. 2C shows the result obtained regarding the plasmid sample D816V 1%. The horizontal axis indicates the temperature (°C) at the time of the measurement. The vertical axis indicates the change in fluorescence intensity (hereinafter also referred to as the "amount of change in fluorescence"), and the unit thereof is "d amount of change in fluorescence intensity/dt" (dF/dt), which is a differential value of the amount of change in fluorescence intensity. The Tm value of a hybrid of the D816V mutant probe 2 with WT is around 48°C, and the Tm value of a hybrid of the D816V mutant probe 2 with D816V is around 56°C.

As can be seen from FIG. 2A, when the plasmid sample WT 100% was used, a peak was observed only at the Tm value of WT. On the other hand, as can be seen from FIGs. 2B and 2C, when the plasmid samples D816V 0.3% and D816V 1% containing both the wild-type plasmid and the mutant plasmid were used, peaks were observed at both the Tm values of WT and D816V. These results demonstrate that, by using the mutant probe, wild-type F primer, mutant F primer, and wild-type R primer according to the present example, even in the case where a wild-type polymorphism and a trace amount of mutant-type polymorphism are present together, it is possible to discriminately detect the wild-type polymorphism and the mutant polymorphism.

### [Example 3]

In the present example, Tm analysis was carried out in the presence of a wild-type plasmid and a mutant plasmid to detect the 822 polymorphism in a c-kit gene.

The wild-type plasmid (WT) and the N822K mutant plasmid (N822K) obtained through insertion of the oligonucleotide having N822K mutant used in Example 1 were mixed together at the predetermined proportion shown in the following table. Thus, three kinds of plasmid samples were prepared.
The plasmid content in each plasmid sample was set to 2 x 10⁴ copies/µl.

**[Table 6]**

| (Plasmid Sample) Plasmid sample | Mixed ratio of respective plasmids | |
|---|---|---|
| | WT | N822K |
| WT 100% | 100% | 0% |
| N822K 0.3% | 99.7% | 0.3% |
| N822K 10% | 90% | 10% |

PCR and Tm analysis were conducted in the same manner as in Example 2, except that the above-described plasmid samples and the following primer and probe were used and conditions for the PCR were set as follows. PCR was conducted in the following manner. The PCR reaction solution was first treated at 95°C for 60 seconds, then was subjected to 50 cycles of treatment with a treatment at 95°C for 1 second and at 63°C for 15 seconds as one cycle, and further was treated at 95°C for 1 second and 40°C for 60 seconds.

The sequences of the wild-type F primer and mutant F primer are shown below. As the R primer, the same primer as in Example 2 was used.

### (wild-type F primer)

F3-wt2 (SEQ ID NO: 18)
   5'-gatcgcagagacatcaagaatgattctaat-3'

### (mutant F primer)

F3-mt2g (SEQ ID NO: 19)
   5'-tggcacagagacatcaagaatgattctaag-3'

As the probe, the following N822K mutant probe 2 was used. The following probe shows a perfect match with a detection target sequence in the antisense strand of the N822K mutant c-kit gene. In the sequence, the underlined base is the base complementary to the N822K mutant. The 5' end of the N822K mutant probe 2 was labeled with a fluorescent dye "TAMRA", and the 3' end of the N822K mutant probe 2 was phosphorylated.
5'-(TAMRA)-ctaagtatgtggttaaaggaaa-P-3' (SEQ ID NO: 9)

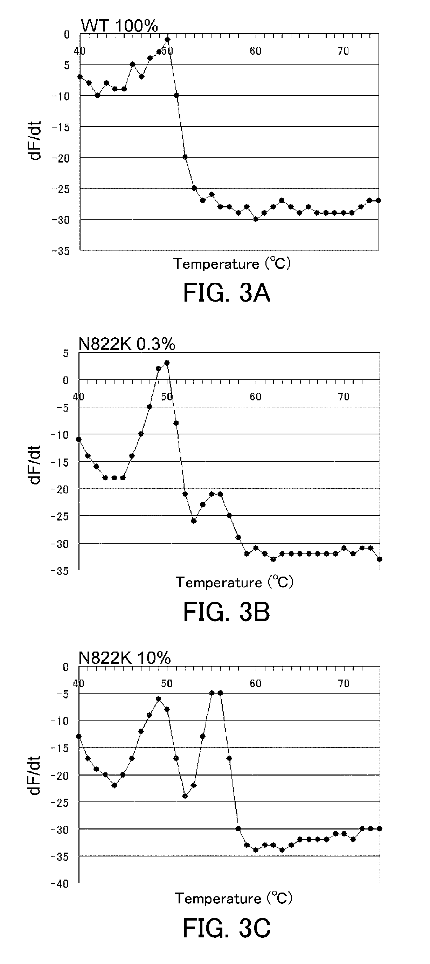

The results are shown in FIG. 3. FIG. 3 show graphs each showing the results of Tm analysis, indicating the change in fluorescence intensity accompanying the temperature rise. FIG. 3A shows the result obtained regarding the plasmid sample WT 100%; FIG. 3B shows the result obtained regarding the plasmid sample N822K 0.3%; and FIG. 3C shows the result obtained regarding the plasmid sample N822K 10%. The horizontal axis indicates the temperature (°C) at the time of the measurement. The vertical axis indicates the change in fluorescence intensity (hereinafter also referred to as the "amount of change in fluorescence"), and the unit thereof is "d amount of change in fluorescence intensity/dt" (dF/dt), which is a differential value of the amount of change in fluorescence intensity. The Tm value of a hybrid of the N822K mutant probe 2 with WT was around 50C°, and the Tm value of a hybrid of the N822K mutant probe 2 with N822K was around 55C°.

As can be seen from FIG. 3A, when the plasmid sample WT 100% was used, a peak was observed only at the Tm value of WT. On the other hand, as can be seen from FIGs. 3B and 3C, when the plasmid samples N822K 0.3% and N822K 10% containing both the wild-type plasmid and the mutant plasmid were used, peaks were observed both in the vicinity of the Tm value of WT and at the Tm value of N822K. These results demonstrate that, by using the mutant probe, wild-type F primer, mutant F primer, and wild-type R primer according to the present example, even in the case where a wild-type polymorphism and a trace amount of mutant-type polymorphism are present together, it is possible to discriminately detect the wild-type polymorphism and the mutant polymorphism.

### [Comparative Example]

In the present example, the 816 polymorphism in a c-kit gene was detected in the same manner as in Example 2, except that the following D816V mutant probes 3 and 4 were used as the probes.

The following probe 3 shows a perfect match with a detection target sequence in the sense strand of the D816V mutant c-kit gene. In the sequence, the underlined base is the base complementary to the D816V mutant. The following probe 4 shows a perfect match with a detection target sequence in the antisense strand of the D816V mutant c-kit gene. In the sequence, the underlined base is the base complementary to the D816V mutant. In each of the probes 3 and 4, the 5' end was labeled with a fluorescent dye "TAMRA", and the 3' end was phosphorylated.
Probe 3 (SEQ ID NO: 21)
   5'-(TAMRA)-cttgatgactctggct-P-3'
Probe 4 (SEQ ID NO: 22)
   5'-(TAMRA)-ctagccagagtcatcaa-P-3'

In the present example, when the plasmid sample WT 100% was used, a peak was observed only at the Tm value of WT. However, when the plasmid samples D816V 0.3% and D816V 1% containing both the wild-type plasmid and the mutant plasmid were used, no peak was observed either at the Tm value of D816V or at the Tm value of D816. As described above, in the case where the mutant probes according to the present comparative example were used, the 816 polymorphism could not be detected.

### Industrial Applicability

As specifically described above, according to the polymorphism detection probe of the present invention, a polymorphism in a c-kit gene can be identified easily with high reliability by Tm analysis, for example. Specifically, for example, even in the case where a c-kit gene having a wild-type target polymorphism and a c-kit gene having a mutant target polymorphism are present together in a sample, the kind of polymorphism or the presence or absence of a mutation can be detected easily with high reliability by Tm analysis using the probe of the present invention. Therefore, the present invention is particularly useful when applied to a sample containing both a wild-type c-kit gene and a mutant c-kit gene. Thus, according to the present invention, a polymorphism in a c-kit gene can be identified easily with high reliability, so that, for example, the detection result can be reflected in the diagnosis of the above-described diseases, the selection of treatment methods for the diseases, and the like. Hence, it can be said that the present invention is very useful in the medical field and the like.

## Claims

1. A probe for detecting a polymorphism in a c-kit gene, the probe comprising.
any one of the following oligonucleotides (P1) to (P4) and (P1') to (P4'):
(P1) a 4- to 50-mer oligonucleotide having a base sequence complementary to a base sequence including the 105th to 108th bases in SEQ ID NO: 1 and having the base complementary to the 105th base in its 3' end region;
(P1') an oligonucleotide having a base sequence complementary to oligonucleotide (P1);
(P2) a 7- to 50-mer oligonucleotide having a base sequence including the 102nd to 108th bases in SEQ ID NO: 1 and having the 102nd base in its 5' end region;
(P2') an oligonucleotide having a base sequence complementary to oligonucleotide (P2);
(P3) an 8- to 50-mer oligonucleotide having a base sequence complementary to a base sequence including the 127th to 134th bases in SEQ ID NO: 1 and having the base complementary to the 134th base in its 5' end region;
(P3') an oligonucleotide having a base sequence complementary to oligonucleotide (P3);
(P4) a 5- to 50-mer oligonucleotide having a base sequence including the 123rd to 127th bases in SEQ ID NO: 1 and having the 123rd base in its 5' end region; and
(P4') an oligonucleotide having a base sequence complementary to oligonucleotide (P4).

2. The probe according to claim 1, wherein the probe comprising any one of the oligonucleotides (P1), (P1'), (P2), and (P2') is a probe for detecting a polymorphism at the 108th base in SEQ ID NO: 1.

3. The probe according to claim 1, wherein the probe comprising any one of the oligonucleotides (P3), (P3'), (P4) and (P4') is a probe for detecting a polymorphism at the 127th base in SEQ ID NO: 1.

4. The probe according to claim 1, wherein
the oligonucleotide (P1) has the base complementary to the 105th base at a position of the 1st to 4th bases from its 3' end,
the oligonucleotide (P2) has the 102nd base at a position of the 1st to 4th bases from its 5' end,
the oligonucleotide (P3) has the base complementary to the 134th base at a position of the 1st to 4th bases from its 5' end, and
the oligonucleotide (P4) has the 123rd base at a position of the 1st to 4th bases from its 5' end.

5. The probe according to claim 1, wherein
the oligonucleotide (P1) has the base complementary to the 105th base at its 3' end,
the oligonucleotide (P2) has the 102nd base at its 5' end,
the oligonucleotide (P3) has the base complementary to the 134th base at its 5' end, and
the oligonucleotide (P4) has the 123rd base at its 5' end.

6. The probe according to claim 1, wherein
the oligonucleotide (P1) is an oligonucleotide shown in SEQ ID NO: 2,
the oligonucleotide (P2) is an oligonucleotide shown in SEQ ID NO: 3,
the oligonucleotide (P3) is an oligonucleotide shown in SEQ ID NO: 4,
and
the oligonucleotide (P4) is an oligonucleotide shown in SEQ ID NO: 5:
5'-aatcattcttgatgwctc-3' (SEQ ID NO: 2)
5'-ccagagwcatcaagaatg-3' (SEQ ID NO: 3)
5'-ccacatahttagaatcattctt-3' (SEQ ID NO: 4)
5'-ctaadtatgtggttaaaggaaa-3' (SEQ ID NO: 5).

7. The probe according to claim 6, wherein
the oligonucleotide (P1) is the oligonucleotide shown in SEQ ID NO: 6,
the oligonucleotide (P2) is the oligonucleotide shown in SEQ ID NO: 7,
the oligonucleotide (P3) is the oligonucleotide shown in SEQ ID NO: 8,
and
the oligonucleotide (P4) is the oligonucleotide shown in SEQ ID NO: 9:
5'-aatcattcttgatgactc-3' (SEQ ID NO: 6)
5'-ccagagtcatcaagaatg-3' (SEQ ID NO: 7)
5'-ccacatacttagaatcattctt-3' (SEQ ID NO: 8)
5'-ctaagtatgtggttaaaggaaa-3' (SEQ ID NO: 9).

8. The probe according to claim 1, wherein the probe is a labeled probe having a fluorescent labeling substance.

9. The probe according to claim 1, wherein the probe is for use in Tm analysis.

10. A method for detecting a polymorphism in a c-kit gene, the method comprising the step of:
detecting a polymorphism in a c-kit gene using the probe according to claim 1.

11. The method according to claim 10, comprising the following steps (A) and (B):
(A) while changing the temperature of a reaction system containing the probe according to claim 1 and a test nucleic acid including the polymorphism to be detected, measuring a signal value indicating the melting state of a hybrid of the test nucleic acid and the probe; and
(B) detecting the polymorphism in the test nucleic acid based on the change in signal value accompanying the temperature change.

12. The method according to claim 11, wherein the reaction system contains two or more kinds of the probes.

13. The method according to claim 12, wherein the probes are a probe composed of either one of the oligonucleotides (P1) and (P2) and a probe composed of either one of the oligonucleotides (P3) and (P4).

14. The method according to claim 12, wherein the two or more kinds of probes are labeled probes having fluorescent labeling substances that are different from each other.

15. The method according to claim 11, wherein,
in step (A), the test nucleic acid is an amplification product obtained by amplifying a template nucleic acid.

16. The method according to claim 15, further comprising the following step (X):
(X) producing an amplification product from a template nucleic acid.

17. The method according to claim 16, wherein
step (X) is the step of producing the amplification product from the template nucleic acid in the reaction system in the presence of the probe, and
in step (A), the signal value is measured while changing the temperature of the reaction system used in step (X).

18. The method according to claim 16, wherein, in step (X), the amplification product is produced using a primer for amplifying a region including at least one of the 108th base and the 127th base in the base sequence of SEQ ID NO: 1.

19. The method according to claim 18, wherein the primer is composed of the following oligonucleotide (F2):
(F2) a 10- to 50-mer oligonucleotide whose 3' end is the 108th base w in the base sequence of SEQ ID NO: 1.

20. The method according to claim 19, wherein the oligonucleotide (F2) is either one of the following oligonucleotides (F2-wt) and (F2-mt):
(F2-wt) a 10- to 50-mer oligonucleotide whose 3' end is the 108th base w in the base sequence of SEQ ID NO: 1 and in which the base w is adenine; and
(F2-mt) a 10- to 50-mer oligonucleotide whose 3' end is the 108th base w in the base sequence of SEQ ID NO: 1 and in which the base w is thymine.

21. The method according to claim 18, wherein the primer comprises: a forward primer composed of either one of the oligonucleotides shown in SEQ ID NOs: 13 and 14; and a reverse primer composed of the oligonucleotide shown in SEQ ID NO: 12:
5'-attttggtctagccagaga-3' (SEQ ID NO: 13)
5'-tgattttggtctagccagagt-3' (SEQ ID NO: 14)
5'-aaatcctttgcaggactgtc-3' (SEQ ID NO: 12).

22. The method according to claim 18, wherein the primer is composed of the following oligonucleotide (F3):
(F3) a 10- to 50-mer oligonucleotide whose 3' end is the 127th base d in the base sequence of SEQ ID NO: 1.

23. The method according to claim 22, wherein the oligonucleotide (F3) is either one of the following oligonucleotides (F3-wt) and (F3-mt):
(F3-wt) a 10- to 50-mer oligonucleotide whose 3' end is the 127th base d in the base sequence of SEQ ID NO: 1 and in which the base d is thymine; and
(F3-mt) a 10- to 50-mer oligonucleotide whose 3' end is the 127th base d in the base sequence of SEQ ID NO: 1 and in which the base d is guanine or adenine.

24. The method according to claim 18, wherein the primer comprises: a forward primer composed of either one of the oligonucleotides shown in SEQ ID NOs: 18 and 19; and a reverse primer composed of the oligonucleotide shown in SEQ ID NO: 12:
5'-gatcgcagagacatcaagaatgattctaat-3' (SEQ ID NO: 18)
5'-tggcacagagacatcaagaatgattctaag-3' (SEQ ID NO: 19)
5'-aaatcctttgcaggactgtc-3' (SEQ ID NO: 12).

25. A primer for amplifying a c-kit gene, the primer being composed of either one of the following oligonucleotides (F2) and (F3):
(F2) a 10- to 50-mer oligonucleotide whose 3' end is the 108th base w in a base sequence of SEQ ID NO: 1; and
(F3) a 10- to 50-mer oligonucleotide whose 3' end is the 127th base d in the base sequence of SEQ ID NO: 1.

26. The primer according to claim 25, wherein the oligonucleotide (F2) is either one of the following oligonucleotides (F2-wt) and (F2-mt):
(F2-wt) a 10- to 50-mer oligonucleotide whose 3' end is the 108th base w in the base sequence of SEQ ID NO: 1 and in which the base w is adenine; and (F2-mt) a 10- to 50-mer oligonucleotide whose 3' end is the 108th base w in the base sequence of SEQ ID NO: 1 and in which the base w is thymine.

27. The primer according to claim 26, wherein
the oligonucleotide (F2-wt) is the oligonucleotide shown in SEQ ID NO: 13, and
the oligonucleotide (F2-mt) is the oligonucleotide shown in SEQ ID NO: 14:
5'-attttggtctagccagaga-3' (SEQ ID NO: 13)
5'-tgattttggtctagccagagt-3' (SEQ ID NO: 14).

28. The primer according to claim 25, wherein the oligonucleotide (F3) is either one of the following oligonucleotides (F3-wt) and (F3-mt):
(F3-wt) a 10- to 50-mer oligonucleotide whose 3' end is the 127th base d in the base sequence of SEQ ID NO: 1 and in which the base d is thymine; and
(F3-mt) a 10- to 50-mer oligonucleotide whose 3' end is the 127th base d in the base sequence of SEQ ID NO: 1 and in which the base d is guanine or adenine.

29. The primer according to claim 28, wherein
the oligonucleotide (F3-wt) is the oligonucleotide shown in SEQ ID NO: 18, and
the oligonucleotide (F3-mt) is the oligonucleotide shown in SEQ ID NO: 19:
5'-gatcgcagagacatcaagaatgattctaat-3' (SEQ ID NO: 18)
5'-tggcacagagacatcaagaatgattctaag-3' (SEQ ID NO: 19).

30. A polymorphism detection reagent comprising:
the probe for detecting a polymorphism in a c-kit gene according to claim 1.

31. The polymorphism detection reagent according to claim 30, further comprising:
a primer for amplifying a region including at least one of the 108th base and the 127th base in the base sequence of SEQ ID NO: 1.

32. The polymorphism detection reagent according to claim 31, wherein the primer is the primer according to claim 25.

33. The polymorphism detection reagent according to claim 31, wherein the primer comprises a forward primer composed of either one of the oligonucleotides shown in SEQ ID NOs: 13 and 14 and a reverse primer composed of the oligonucleotide shown in SEQ ID NO: 12:
5'-attttggtctagccagaga-3' (SEQ ID NO: 13)
5'-tgattttggtctagccagagt-3' (SEQ ID NO: 14)
5'-aaatcctttgcaggactgtc-3' (SEQ ID NO: 12).

34. The polymorphism detection reagent according to claim 31, wherein the primer comprises: a forward primer composed of either one of the oligonucleotides shown in SEQ ID NOs: 18 and 19; and a reverse primer composed of the oligonucleotide shown in SEQ ID NO: 12:
5'-gatcgcagagacatcaagaatgattctaat-3' (SEQ ID NO: 18)
5'-tggcacagagacatcaagaatgattctaag-3' (SEQ ID NO: 19)
5'-aaatcctttgcaggactgtc-3' (SEQ ID NO: 12).

## Amended claims

### Amended claims under Art. 19.1 PCT

**1.** Amended) A probe for detecting a polymorphism in a c-kit gene, the probe comprising:
any one of the following oligonucleotides (P1) to (P4) and (P1') to (P4'):
(P1) a 4- to 50-mer oligonucleotide having a base sequence complementary to a base sequence including the 105th to 108th bases in SEQ ID NO: 1 and in which the base complementary to the 105th base is labeled with a fluorescent dye and is in its 3' end region;
(P2) a 7- to 50-mer oligonucleotide having a base sequence including the 102nd to 108th bases in SEQ ID NO: 1 and in which the 102nd base is labeled with a fluorescent dye and is in its 5' end region;
(P3) an 8- to 50-mer oligonucleotide having a base sequence complementary to a base sequence including the 127th to 134th bases in SEQ ID NO: 1 and in which the base complementary to the 134th base is labeled with a fluorescent dye and is in its 5' end region; and
(P4) a 5- to 50-mer oligonucleotide having a base sequence including the 123rd to 127th bases in SEQ ID NO: 1 and in which the 123rd base is labeled with a fluorescent dye and is in its 5' end region.

**2.** Cancelled)

**3.** (Cancelled)

**4.** The probe according to claim 1, wherein
the oligonucleotide (P1) has the base complementary to the 105th base at a position of the 1st to 4th bases from its 3' end,
the oligonucleotide (P2) has the 102nd base at a position of the 1st to 4th bases from its 5' end,
the oligonucleotide (P3) has the base complementary to the 134th base at a position of the 1st to 4th bases from its 5' end, and
the oligonucleotide (P4) has the 123rd base at a position of the 1st to 4th bases from its 5' end.

**5.** The probe according to claim 1, wherein
the oligonucleotide (P1) has the base complementary to the 105th base at its 3' end,
the oligonucleotide (P2) has the 102nd base at its 5' end,
the oligonucleotide (P3) has the base complementary to the 134th base at its 5' end, and
the oligonucleotide (P4) has the 123rd base at its 5' end.

**6.** The probe according to claim 1, wherein
the oligonucleotide (P1) is an oligonucleotide shown in SEQ ID NO: 2,
the oligonucleotide (P2) is an oligonucleotide shown in SEQ ID NO: 3,
the oligonucleotide (P3) is an oligonucleotide shown in SEQ ID NO: 4, and
the oligonucleotide (P4) is an oligonucleotide shown in SEQ ID NO: 5:
5'-aatcattcttgatgwctc-3' (SEQ ID NO: 2)
5'-ccagagwcatcaagaatg-3' (SEQ ID NO: 3)
5'-ccacatahttagaatcattctt-3' (SEQ ID NO: 4)
5'-ctaadtatgtggttaaaggaaa-3' (SEQ ID NO: 5).

**7.** The probe according to claim 6, wherein
the oligonucleotide (P1) is the oligonucleotide shown in SEQ ID NO: 6,
the oligonucleotide (P2) is the oligonucleotide shown in SEQ ID NO: 7,
the oligonucleotide (P3) is the oligonucleotide shown in SEQ ID NO: 8, and
the oligonucleotide (P4) is the oligonucleotide shown in SEQ ID NO: 9:
5'-aatcattcttgatgactc-3' (SEQ ID NO: 6)
5'-ccagagtcatcaagaatg-3' (SEQ ID NO: 7)
5'-ccacatacttagaatcattctt-3' (SEQ ID NO: 8)
5'-ctaagtatgtggttaaaggaaa-3' (SEQ ID NO: 9).

**8.** Cancelled)

**9.** The probe according to claim 1, wherein the probe is for use in Tm analysis.

**10.** A method for detecting a polymorphism in a c-kit gene, the method comprising the step of:
detecting a polymorphism in a c-kit gene using the probe according to claim 1.

**11.** The method according to claim 10, comprising the following steps (A) and (B):
(A) while changing the temperature of a reaction system containing the probe according to claim 1 and a test nucleic acid including the polymorphism to be detected, measuring a signal value indicating the melting state of a hybrid of the test nucleic acid and the probe; and
(B) detecting the polymorphism in the test nucleic acid based on the change in signal value accompanying the temperature change.

**12.** The method according to claim 11, wherein the reaction system contains two or more kinds of the probes.

**13.** The method according to claim 12, wherein the probes are a probe composed of either one of the oligonucleotides (P1) and (P2) and a probe composed of either one of the oligonucleotides (P3) and (P4).

**14.** The method according to claim 12, wherein the two or more kinds of probes are labeled probes having fluorescent labeling substances that are different from each other.

**15.** The method according to claim 11, wherein,
in step (A), the test nucleic acid is an amplification product obtained by amplifying a template nucleic acid.

**16.** The method according to claim 15, further comprising the following step (X):
(X) producing an amplification product from a template nucleic acid.

**17.** The method according to claim 16, wherein
step (X) is the step of producing the amplification product from the template nucleic acid in the reaction system in the presence of the probe, and
in step (A), the signal value is measured while changing the temperature of the reaction system used in step (X).

**18.** The method according to claim 16, wherein, in step (X), the amplification product is produced using a primer for amplifying a region including at least one of the 108th base and the 127th base in the base sequence of SEQ ID NO: 1.

**19.** The method according to claim 18, wherein the primer is composed of the following oligonucleotide (F2):
(F2) a 10- to 50-mer oligonucleotide whose 3' end is the 108th base w in the base sequence of SEQ ID NO: 1.

**20.** The method according to claim 19, wherein the oligonucleotide (F2) is either one of the following oligonucleotides (F2-wt) and (F2-mt):
(F2-wt) a 10- to 50-mer oligonucleotide whose 3' end is the 108th base w in the base sequence of SEQ ID NO: 1 and in which the base w is adenine; and
(F2-mt) a 10- to 50-mer oligonucleotide whose 3' end is the 108th base w in the base sequence of SEQ ID NO: 1 and in which the base w is thymine.

**21.** The method according to claim 18, wherein the primer comprises: a forward primer composed of either one of the oligonucleotides shown in SEQ ID NOs: 13 and 14; and a reverse primer composed of the oligonucleotide shown in SEQ ID NO: 12:
5'-attttggtctagccagaga-3' (SEQ ID NO: 13)
5'-tgattttggtctagccagagt-3' (SEQ ID NO: 14)
5'-aaatcctttgcaggactgtc-3' (SEQ ID NO: 12).

**22.** The method according to claim 18, wherein the primer is composed of the following oligonucleotide (F3):
(F3) a 10- to 50-mer oligonucleotide whose 3' end is the 127th base d in the base sequence of SEQ ID NO: 1.

**23.** The method according to claim 22, wherein the oligonucleotide (F3) is either one of the following oligonucleotides (F3-wt) and (F3-mt):
(F3-wt) a 10- to 50-mer oligonucleotide whose 3' end is the 127th base d in the base sequence of SEQ ID NO: 1 and in which the base d is thymine; and
(F3-mt) a 10- to 50-mer oligonucleotide whose 3' end is the 127th base d in the base sequence of SEQ ID NO: 1 and in which the base d is guanine or adenine.

**24.** The method according to claim 18, wherein the primer comprises: a forward primer composed of either one of the oligonucleotides shown in SEQ ID NOs: 18 and 19; and a reverse primer composed of the oligonucleotide shown in SEQ ID NO: 12:
5'-gatcgcagagacatcaagaatgattctaat-3' (SEQ ID NO: 18)
5'-tggcacagagacatcaagaatgattctaag-3' (SEQ ID NO: 19)
5'-aaatcctttgcaggactgtc-3' (SEQ ID NO: 12).

**25.** Cancelled)

**26.** (Cancelled)

**27.** Cancelled)

**28.** (Cancelled)

**29.** Cancelled)

**30.** A polymorphism detection reagent comprising:
the probe for detecting a polymorphism in a c-kit gene according to claim 1.

**31.** The polymorphism detection reagent according to claim 30, further comprising:
a primer for amplifying a region including at least one of the 108th base and the 127th base in the base sequence of SEQ ID NO: 1.

**32.** Cancelled)

**33.** (Cancelled)

**34.** Cancelled)


### [Sequence Listing]

SEQUENCE LISTING

<110>  Arkray, Inc.,

<120>  Probe for detecting of polymorphism of c-kit gene and use thereof

<130>  TF10064-01WO

<150>  JP 2009-293278
<151>  2009-12-24

<160>  25

<170>  PatentIn version 3.1

<210>  1
<211>  240
<212>  DNA
<213>  Homo sapiens

<400>  1
cttttctcct ccaacctaat agtgtattca cagagacttg gcagccagaa atatcctcct      60

tactcatggt cggatcacaa agatttgtga ttttggtcta gccagagwca tcaagaatga     120

ttctaadtat gtggttaaag gaaacgtgag tacccattct ctgcttgaca gtcctgcaaa     180

ggatttttag tttcaacttt cgataaaaat tgtttcctgt gattttcata atgtaaatcc     240


<210>  2
<211>  18
<212>  DNA
<213>  Artificial

<220>
<223>  probe

<400>  2
aatcattctt gatgwctc                                                    18


<210>  3
<211>  18
<212>  DNA
<213>  Artificial

<220>
<223>  probe

<400>  3
ccagagwcat caagaatg                                                    18


<210>  4
<211>  22
<212>  DNA
<213>  Artificial

<220>
<223>  probe

<400>  4
ccacatahtt agaatcattc tt                                               22

<210>  5
<211>  22
<212>  DNA
<213>  Artificial

<220>
<223>  probe

<400>  5
ctaadtatgt ggttaaagga aa                                                22


<210>  6
<211>  18
<212>  DNA
<213>  Artificial

<220>
<223>  probe

<400>  6
aatcattctt gatgactc                                                     18


<210>  7
<211>  18
<212>  DNA
<213>  Artificial

<220>
<223>  probe

<400>  7
ccagagtcat caagaatg                                                     18


<210>  8
<211>  22
<212>  DNA
<213>  Artificial

<220>
<223>  probe

<400>  8
ccacatactt agaatcattc tt                                                22


<210>  9
<211>  22
<212>  DNA
<213>  Artificial

<220>
<223>  probe

<400>  9
ctaagtatgt ggttaaagga aa                                                22


<210>  10
<211>  21
<212>  DNA
<213>  Artificial

<220>
<223>  primer

<400>  10
tgtattcaca gagacttggc a                                                21


<210>  11
<211>  21
<212>  DNA
<213>  Artificial

<220>
<223>  primer

<400>  11
gagaatgggt actcacgttt c                                                21


<210>  12
<211>  20
<212>  DNA
<213>  Artificial

<220>
<223>  primer

<400>  12
aaatcctttg caggactgtc                                                  20


<210>  13
<211>  19
<212>  DNA
<213>  Artificial

<220>
<223>  primer

<400>  13
attttggtct agccagaga                                                   19


<210>  14
<211>  21
<212>  DNA
<213>  Artificial

<220>
<223>  primer

<400>  14
tgattttggt ctagccagag t                                                21


<210>  15
<211>  25
<212>  DNA
<213>  Artificial

<220>
<223>  primer

<400>  15

cagagacatc aagaatgatt ctaat                                          25

<210>  16
<211>  25
<212>  DNA
<213>  Artificial

<220>
<223>  primer

<400>  16
cagagacatc aagaatgatt ctaag                                          25

<210>  17
<211>  25
<212>  DNA
<213>  Artificial

<220>
<223>  primer

<400>  17
cagagacatc aagaatgatt ctaaa                                          25

<210>  18
<211>  30
<212>  DNA
<213>  Artificial

<220>
<223>  primer

<400>  18
gatcgcagag acatcaagaa tgattctaat                                     30

<210>  19
<211>  30
<212>  DNA
<213>  Artificial

<220>
<223>  primer

<400>  19
tggcacagag acatcaagaa tgattctaag                                     30

<210>  20
<211>  30
<212>  DNA
<213>  Artificial

<220>
<223>  primer

<400>  20
tggcacagag acatcaagaa tgattctaaa                                     30

<210>  21
<211>  16

<212>  DNA
<213>  Artificial

<220>
<223>  probe

<400>  21
cttgatgact ctggct                                                    16


<210>  22
<211>  17
<212>  DNA
<213>  Artificial

<220>
<223>  probe

<400>  22
ctagccagag tcatcaa                                                   17


<210>  23
<211>  300
<212>  DNA
<213>  Artificial

<220>
<223>  oligonucleotide

<400>  23
cactatagta ttaaaaagtt agttttcact ctttacaagt taaaatgaat ttaaatggtt      60

ttcttttctc ctccaaccta atagtgtatt cacagagact tggcagccag aaatatcctc     120

cttactcatg gtcggatcac aaagatttgt gattttggtc tagccagaga catcaagaat     180

gattctaatt atgtggttaa aggaaacgtg agtacccatt ctctgcttga cagtcctgca     240

aaggattttt agtttcaact ttcgataaaa attgtttcct gtgactttca taatgtaaat     300


<210>  24
<211>  300
<212>  DNA
<213>  Artificial

<220>
<223>  oligonucleotide

<400>  24
cactatagta ttaaaaagtt agttttcact ctttacaagt taaaatgaat ttaaatggtt      60

ttcttttctc ctccaaccta atagtgtatt cacagagact tggcagccag aaatatcctc     120

cttactcatg gtcggatcac aaagatttgt gattttggtc tagccagagt catcaagaat     180

gattctaatt atgtggttaa aggaaacgtg agtacccatt ctctgcttga cagtcctgca     240

aaggattttt agtttcaact ttcgataaaa attgtttcct gtgactttca taatgtaaat     300


<210>  25
<211>  300
<212>  DNA

<213>  Artificial

<220>
<223>  oligonucleotide

<400>  25
cactatagta ttaaaaagtt agttttcact ctttacaagt taaaatgaat ttaaatggtt      60

ttcttttctc ctccaaccta atagtgtatt cacagagact tggcagccag aaatatcctc     120

cttactcatg gtcggatcac aaagatttgt gattttggtc tagccagaga catcaagaat     180

gattctaagt atgtggttaa aggaaacgtg agtacccatt ctctgcttga cagtcctgca     240

aaggattttt agtttcaact ttcgataaaa attgtttcct gtgactttca taatgtaaat     300